# EUROPÄISCHE PATENTSCHRIFT

(11) **EP 4 355 717 B1**
(45) Veröffentlichungstag und Bekanntmachung des Hinweises auf die Patenterteilung: **07.05.2025**
(21) Anmeldenummer: 22717154.3
(22) Anmeldetag: 23.03.2022
(51) Int. Cl.: C07C 29/80, C07C 31/04, C07C 29/70, C07C 31/30, B01D 3/14

(54) **VERFAHREN ZUR AUFARBEITUNG EINES METHANOL/WASSER-GEMISCHES BEI DER HERSTELLUNG VON ALKALIMETALLMETHANOLATEN IN EINER REAKTIONSKOLONNE**
PROCESS FOR WORKUP OF A METHANOL/WATER MIXTURE IN THE PRODUCTION OF ALKALI METAL METHOXIDES IN A REACTION COLUMN
PROCÉDÉ DE TRAITEMENT D'UN MÉLANGE MÉTHANOL/EAU DANS LA PRODUCTION DE MÉTHOXYDES DE MÉTAUX ALCALINS DANS UNE COLONNE DE RÉACTION

(30) Priorität: 16.06.2021 EP 21179722
(43) Veröffentlichungstag der Anmeldung: 24.04.2024
(73) Patentinhaber: Evonik Operations GmbH, 45128 Essen (DE)
(72) Erfinder: ROETTGER, Dirk, 50672 Köln (DE); SCHRÖDER, Moritz, 48153 Münster (DE); PAUL, Niklas, 45770 Marl (DE); RIX, Armin Matthias, 45770 Marl (DE); ZITZEWITZ, Philip, 45721 Haltern am See (DE); OLDEMEYER, Martin, 45721 Haltern am See (DE); WÜLLER, Martin, 45768 Marl (DE); SIX, Tanita Valèrie, 44309 Dortmund (DE); RUWWE, Johannes, 63457 Hanau (DE)
(74) Vertreter: Evonik Patent Association
(86) Internationale Anmeldenummer: PCT/EP2022/057591
(87) Internationale Veröffentlichungsnummer: WO 2022/263032

(56) Entgegenhaltungen:
- WO-A1-2010/097318
- CN-A- 105 218 315
- US-A- 3 418 383

## Beschreibung

Die vorliegende Erfindung betrifft ein Verfahren zur Aufarbeitung eines Methanol/Wasser-Gemischs, welches bei der Herstellung von Alkalimetallmethanolaten in einer Reaktionskolonne eingesetzt wird. Dabei wird das Gemisch in einer Rektifikationskolonne destillativ getrennt. Die am oberen Ende der Rektifikationskolonne erhaltenen Brüden werden in mindestens zwei Stufen verdichtet und die Energie der jeweils verdichteten Brüden vorteilhaft auf Sumpf- und Seitenstrom der Rektifikationskolonne übertragen. Dies ermöglicht den besonders energieeffizienten Einsatz der Energie der verdichteten Brüden im erfindungsgemäßen Verfahren.

Das Verfahren zur Aufarbeitung eines Methanol/Wasser-Gemischs wird bei der Herstellung von Alkalimetallmethanolaten in einer Reaktionskolonne eingesetzt, wobei in einer Reaktionskolonne Methanol und Alkalilauge im Gegenstrom miteinander umgesetzt werden. Am unteren Ende wird dabei in Methanol gelöstes Alkalimetallmethanolat entnommen, und am oberen Ende wird ein Methanol/Wasser-Gemisch entnommen, welches mit dem erfindungsgemäßen Aufarbeitungsverfahren aufgearbeitet wird. Die Energie der verdichteten Brüden kann außerdem zum Betrieb der Reaktionskolonne eingesetzt werden oder zum Betrieb einer Reaktionskolonne, in welcher ein Verfahren zur Umalkoholisierung von Alkalimetallalkoholaten durchgeführt wird.

### 1. Hintergrund der Erfindung

Alkohol/Wasser-Gemische fallen zum Beispiel bei der Herstellung von Alkalimetallalkoholaten aus wässriger, gegebenenfalls mit Alkohol versetzter Alkalilauge und Alkohol an. In diesen Verfahren werden insbesondere Methanol und Ethanol als Alkohol eingesetzt.

Alkalimetallalkoholate werden als starke Basen in der Synthese zahlreicher Chemikalien, z.B. bei der Herstellung von Pharma- oder Agrowirkstoffen, eingesetzt. Weiterhin finden Alkalimetallalkoholate Anwendung als Katalysatoren in Umesterungs- und Amidierungsreaktionen.

Alkalimetallalkoholate (MOR) werden mittels Reaktivdestillation in einer Gegenstromdestillationskolonne aus Alkalimetallhydroxiden (MOH) und Alkoholen (ROH) hergestellt, wobei das gemäß folgender Reaktion <1> entstehende Reaktionswasser mit dem Destillat entfernt wird.

MOH + ROH 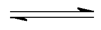 MOR + H₂O.

Ein solches Verfahrensprinzip ist beispielsweise in der US 2,877,274 A beschrieben, wobei wässrige Alkalimetallhydroxid-Lösung und gasförmiges Methanol im Gegenstrom in einer Reaktivrektifikationskolonne gefahren werden. In prinzipiell nicht veränderter Form wird dieses Verfahren in der WO 01/42178 A1 erneut beschrieben.

Ähnliche Verfahren, bei denen jedoch zusätzlich noch ein Schleppmittel, wie beispielsweise Benzol, eingesetzt wird, sind in der GB 377,631 A und der US 1,910,331 A beschrieben. Das Schleppmittel dient hierbei zur Trennung von Wasser und dem wasserlöslichen Alkohol. Bei beiden Patentschriften wird das Kondensat einer Phasentrennung unterzogen, um das Reaktionswasser abzutrennen. Ein weiteres ähnliches Verfahren ist die Umsetzung eines Alkalimetallalkoholats mit einem anderen Alkohol in einer Reaktionskolonne ("Umalkoholisierung") gemäß DE 27 26 491 A1.

Entsprechend beschreibt die DE 96 89 03 C ein Verfahren zur kontinuierlichen Herstellung von Alkalimetallalkoholaten in einer Reaktionskolonne, wobei das am Kopf entnommene Wasser-Alkohol-Gemisch kondensiert und anschließend einer Phasentrennung unterworfen wird. Die wässrige Phase wird hierbei verworfen und die alkoholische Phase wird zusammen mit dem frischen Alkohol der Kolonne am Kopf zurückgegeben. Ein ähnliches Verfahren beschreibt die EP 0 299 577 A2, wobei die Wasserabtrennung im Kondensat mit Hilfe einer Membran erfolgt. Die industriell wichtigsten Alkalimetallalkoholate sind jene des Natriums und Kaliums, und hierbei insbesondere die Methylate und Ethylate. Deren Synthese ist vielfach im Stand der Technik beschrieben, zum Beispiel in der EP 1 997 794 A1.

Bei den im Stand der Technik beschriebenen Synthesen der Alkalimetallalkoholate durch Reaktivrektifikation werden üblicherweise Brüden erhalten, welche den eingesetzten Alkohol und Wasser umfassen. Es ist aus wirtschaftlichen Gründen sinnvoll, den von den Brüden umfassten Alkohol wieder in der Reaktivdestillation als Edukt einzusetzen. Deshalb werden die Brüden üblicherweise einer Rektifikationskolonne zugeführt und der darin enthaltene Alkohol abgetrennt (beschrieben beispielsweise in der GB 737 453 A und der US 4,566,947 A). Der so zurückgewonnene Alkohol wird dann beispielsweise als Edukt der Reaktivdestillation zugeführt.

Alternativ oder zusätzlich kann ein Teil des Alkoholbrüden zur Beheizung der Rektifikationskolonne genutzt werden (beschrieben in WO 2010/097318 A1). Dazu muss der Brüden allerdings verdichtet werden, um das zur Beheizung der Rektifikationskolonne nötige Temperaturniveau zu erreichen. Insbesondere eine mehrstufige Verdichtung des Brüden ist thermodynamisch vorteilhaft. Hierbei wird der Brüden zwischen den Verdichtungsstufen gekühlt. Darüber hinaus trägt die Zwischenkühlung dazu bei, die maximal zulässige Temperatur des Verdichters nicht zu überschreiten. Der Nachteil dieser in den herkömmlichen Verfahren durchgeführten Kühlung ist, dass die dabei entzogene Energie ungenutzt dissipiert.

Es besteht demnach der Bedarf nach verbesserten Verfahren zur Aufarbeitung eines Alkohol/Wasser-Gemischs, was insbesondere im Kontext eines Verfahrens zur Herstellung von Alkalimetallalkoholaten eingesetzt werden kann. Dieses Verfahren soll sich durch eine besonders effiziente Nutzung der im verdichteten Brüden enthaltenen Energie zum Betrieb der Rektifikationskolonne auszeichnen. Das Verfahren soll so eine energieeffiziente Nutzung der bei der Verdichtung und Abkühlung der Brüden anfallenden Wärme ermöglichen.

### 2. Kurzzusammenfassung der Erfindung

Die vorliegende Erfindung betrifft ein Verfahren zur Herstellung mindestens eines Alkalimetallalkoholats der Formel M_{A}OR, wobei R Methyl, ist, und wobei M_{A} ein Metall ausgewählt aus Natrium, Kalium, bevorzugt Natrium ist.

Im erfindungsgemäßen Verfahren zur Herstellung mindestens eines Alkalimetallalkoholats wird ein Gemisch **G** umfassend Wasser und Methanol ROH erhalten, welches in einem Verfahren zur Aufarbeitung eines Gemisches **G** umfassend Wasser und Alkohol ROH, wobei **R** Methyl ist eingesetzt wird und mit dem Verfahren zur Aufarbeitung eines Gemisches **G** aufgearbeitet wird. Das Verfahren zur Aufarbeitung eines Gemisches **G** wird in einer Rektifikationskolonne durchgeführt und ist demnach ein destillatives Verfahren.

In einem weiteren, bevorzugten Aspekt betrifft die vorliegende Erfindung ein Verfahren zur Umalkoholisierung von Alkalimetallalkoholaten. In diesem Verfahren wird der Alkohol eines Alkalimetallalkoholats M_{c}OR' durch einen anderen Alkohol R"OH ausgetauscht, in dem M_{c}OR' mit R"OH in einer Reaktionskolonne zu M_{c}OR" umgesetzt wird, wobei in dem Verfahren Energie von bestimmten Brüdenströmen aus dem erfindungsgemäßen Verfahren zur Aufarbeitung eines Gemisches **G** zum Betrieb genutzt wird.

### 3. Abbildungen

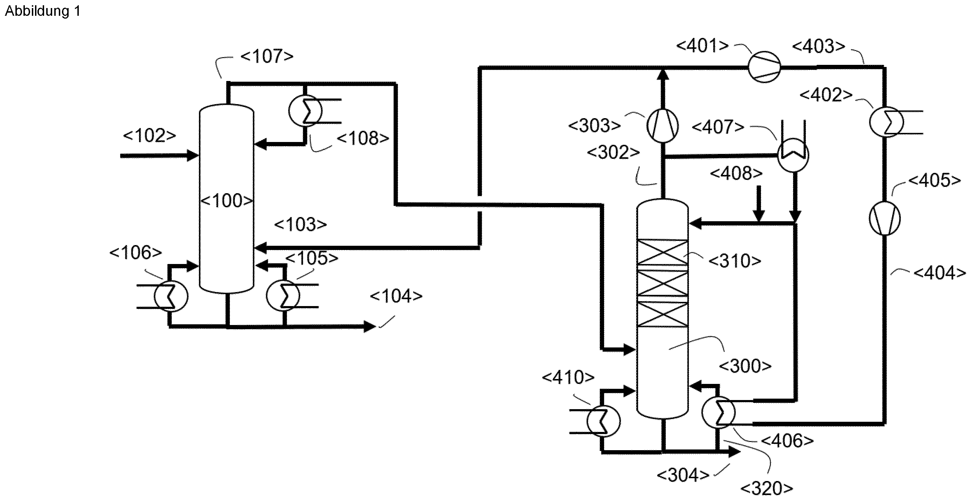

### 3.1 Abbildung 1

Abbildung 1 zeigt eine nicht erfindungsgemäße Ausführungsform eines Verfahrens zur Herstellung von Alkalimetallmethanolaten, in dem die destillative Trennung des Methanol-Wasser-Gemisches wie im Stand der Technik (WO 2010/097318 A1, Abbildung 1) erfolgt.

Dabei wird wässrige NaOH **S_{AE2}** <102> in einer Reaktionskolonne **RR_{A}** <100> mit Methanol **S_{AE1}** <103> zum entsprechenden Natriumalkoholat umgesetzt. Am Kopf der Reaktionskolonne **RR_{A}** <100> wird eine wässrige NaOH-Lösung als Eduktstrom **S_{AE2}** <102> zugegeben. Alternativ kann auch eine methanolische NaOH-Lösung als Eduktstrom **S_{AE2}** <102> zugegeben werden. Zur Herstellung des entsprechenden Kaliummethanolats wird wässrige oder methanolische KOH-Lösung als Eduktstrom **S_{AE2}** <102> zugegeben. Oberhalb des Sumpfes der Reaktionskolonne **RR_{A}** <100> wird Methanol als Eduktstrom **S_{AE1}** <103> dampfförmig zugegeben.

Am Sumpf der Reaktionskolonne **RR_{A}** <100> wird ein Gemisch des entsprechenden Methanolats im Methanol **S_{AP*}** <104> entnommen. Mit dem Sumpfverdampfer **V_{SA}** <105> und dem optionalen Verdampfer **V_{SA'}** <106> am Sumpf der Kolonne **RR_{A}** <100> wird die Konzentration der Natriummethanolatlösung **S_{AP*}** <104> auf den gewünschten Wert eingestellt.

Am Kopf der Reaktionskolonne **RR_{A}** <100> wird ein Brüdenstrom **S_{AB}** <107> entnommen. Im Kondensator **K_{RRA}** <108> wird ein Teil des Brüdenstroms **S_{AB}** <107> kondensiert und flüssig als Rücklauf auf den Kopf der Reaktionskolonne **RR_{A}** <100> aufgegeben. Kondensator **K_{RRA}** <108> und die Einstellung des Rücklaufs sind jedoch optional.

Der erhaltene Brüden **S_{AB}** <107> wird ganz oder teilweise einer Rektifikationskolonne, ener Wasser/Methanol-Kolonne, **RD_{A}** <300> als Gemisch **G** zugeführt. Die Rektifikationskolonne **RD_{A}** <300> enthält Einbauten <310>. In ihr wird das Gemisch **G** destillativ aufgetrennt, und an ihrem Kopf das Methanol als Brüden **S_{OA}** <302> destillativ rückgewonnen.

An der Rektifikationskolonne **RD_{A}** <300> kann ein Rücklauf eingestellt werden. In diesem Fall wird ein Teil des Brüden **S_{OA}** <302> in einem Kondensator **K_{RD}** <407> kondensiert und dann wieder auf die Rektifikationskolonne **RD_{A}** <300> rückgeführt. Der übrige Teil von **S_{OA}** <302>, bzw. der komplette Brüdenstrom **S_{OA}** <302> in den Ausführungsformen, in denen kein Rücklauf eingestellt wird, wird mittels Verdichter **VD_{AB2}** <303> vorverdichtet. Ein Teil dieses vorverdichteten Brüdens wird zur Reaktionskolonne **RR_{A}** <100> rezykliert, wo er als Eduktstrom **S_{AE1}** <103> eingesetzt wird. Der übrige Teil von **S_{OA}** <302> wird dem Verdichter **VD₁** <401> zugeleitet, wo er weiter zum Brüdenstrom **S_{OA1}** <403> verdichtet wird, von dem im optionalen Zwischenkühler **WT_{X}** <402> Energie abgeführt werden kann.

Der Brüdenstrom **S_{OA1}** <403> wird erneut mittels Verdichter **VDₓ** <405> verdichtet und der dann erhaltene Brüden **S_{OA2}** <404> dem Verdampfer **V_{SRD}** <406> am Sumpf der Rektifikationskolonne **RD_{A}** <300> zur Beheizung zugeführt, wonach ihm gegebenenfalls frisches Methanol <408> zugegeben wird und er wieder der Rektifikationskolonne **RD_{A}** <300> als Rücklauf zugeführt wird. Falls ein Rücklauf an der Rektifikationskolonne **RD_{A}** <300> eingestellt wird, kann der Strom **S_{OA2}** <404>, bevor er in **RD_{A}** <300> rückgeleitet wird, mit dem Rücklauf, also dem Kondensat aus **K_{RD}** <407>, vermischt werden und mit diesem zusammen in **RD_{A}** <300> gespeist werden. Am Sumpf der Rektifikationskolonne **RD_{A}** <300> wird ein Wasserstrom **S_{UA}** <304> erhalten, der mindestens teilweise (Strom **S_{UA1}** <320>) wieder in die Rektifikationskolonne **RD_{A}** <300> rückgeführt werden kann, wobei er über den Verdampfer **V_{SRD}** <406> und/oder **V_{SRD'}** <410> geführt werden kann.

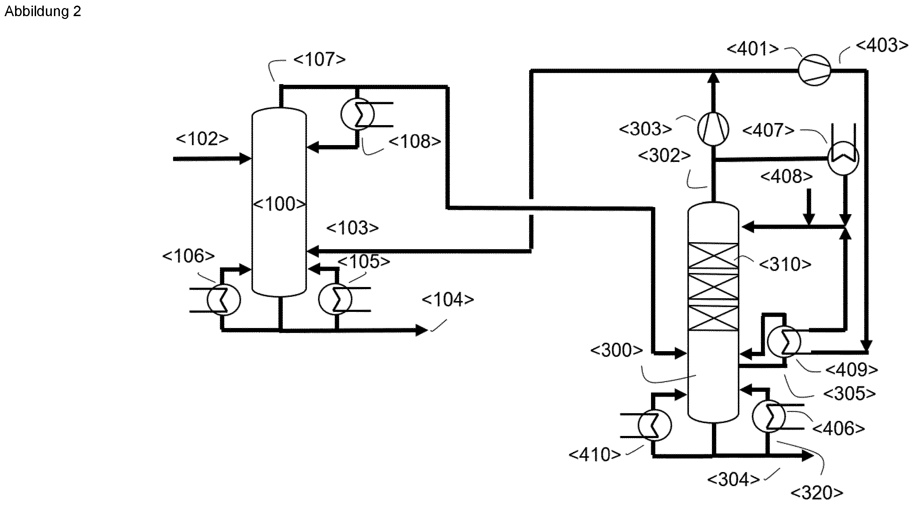

### 3.2 Abbildung 2

Abbildung 2 zeigt eine weitere nicht erfindungsgemäßen Ausführungsform eines Verfahrens, welche der in Abbildung 2 der WO 2010/097318 A1 gezeigten Ausführungsform entspricht.

Diese Ausführungsform entspricht der in Abbildung 1 beschriebenen mit folgenden zusätzlichen bzw. unterschiedlichen Merkmalen: Neben den Verdampfern **V_{SRD'}** <406> und **V_{SRD'}** <410> am Sumpf weist die Rektifikationskolonne **RD_{A}** <300> einen Zwischenverdampfer **V_{ZRD}** <409> auf. Ein Seitenstrom **S_{ZA}** <305> wird der Rektifikationskolonne **RD_{A}** <300> entnommen und über **V_{ZRD}** <409> geführt, danach der Rektifikationskolonne **RD_{A}** <300> wieder zugeführt. Ein Teil des Brüdenstroms **S_{OA} <302>** wird mittels Verdichter **VD_{AB2}** <303> vorverdichtet und, nachdem ein Teil davon zur Reaktionskolonne **RR_{A}** <100> abgezweigt wurde, im Verdichter **VD₁** <401> zum Brüdenstrom **S_{OA1}** <403> verdichtet, und dieser dem Zwischenverdampfer **V_{ZRD}** <409> zur Beheizung zugeführt. Eine Beheizung im Verdampfer **V_{SRD}** <406> oder im Verdampfer **V_{SRD'}** <410> durch **S_{OA1}** <403> erfolgt nicht. Der zur Beheizung von **V_{ZRD}** <409> genutzte Brüdenstrom **S_{OA}** <302> wird danach mit dem Kondensat aus **K_{RD}** <407> und dem frischen Methanol <408> gemischt und als Rücklauf in die Rektifikationskolonne **RD_{A}** <300> zurückgeführt. Eine getrennte Rückführung des Rücklaufs von **S_{OA} <302>** in die Rektifikationskolonne **RD_{A}** <300> ist ebenfalls möglich.

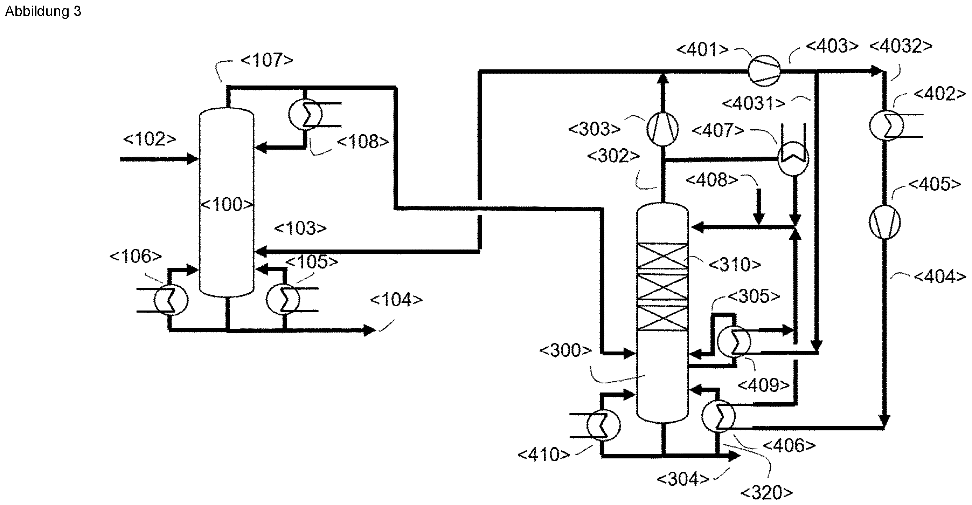

### 3.3 Abbildung 3

Abbildung 3 zeigt eine Ausführungsform des erfindungsgemäßen Verfahrens. Diese entspricht den in Abbildungen 1 und 2 beschriebenen Ausführungsformen. Die Rektifikationskolonne **RD_{A}** <300> weist einen Zwischenverdampfer **V_{ZRD}** <409> und einen Sumpfverdampfer **V_{SRD}** <406> sowie optional den Sumpfverdampfer **V_{SRD'}** <410> auf.

Die erfindungsgemäße Ausführungsform weist folgende Unterschiede zu den vorbeschriebenen Ausführungsformen auf:
1. Nach Verdichtung eines Teils des Brüdenstroms **S_{OA}** <302> im Verdichter **VD₁** <401> wird der Brüdenstrom **S_{OA1}** <403> in zwei Teile **S_{OA11}** <4031> und **S_{OA12}** <4032> geteilt.
2. **S_{OA11}** <4031> wird dem Zwischenverdampfer **V_{ZRD}** <409> zur Beheizung des Stromes **S_{ZA}** <305> zugeführt.
3. **S_{OA12}** <4032> wird weiter im Verdichter **VDₓ** <405> zum Strom **S_{OA2}** <404> verdichtet und **S_{OA2}** <404> dem Sumpfverdampfer **V_{SRD}** <406> zur Beheizung des Stromes **S_{UA1}** <320> zugeführt.
4. Nachdem **S_{OA11}** <4031> und **S_{OA2}** <404> den jeweiligen Verdampfer **V_{ZRD}** <409> bzw. **V_{SRD}** <406> verlassen haben, werden sie vereinigt und der vereinigte Strom mit dem Rücklauf (also dem Kondensat aus **K_{RD}** <407>) und dem frischen Methanol <408> gemischt und in die Rektifikationskolonne **RD_{A}** <300> zurückgeführt. Eine getrennte Rückführung dieser Ströme in die Rektifikationskolonne **RD_{A}** <300> ist ebenfalls möglich.

Aufgrund dieser Unterschiede kann die Energie des Brüdens **S_{OA1}** <403> im Vergleich zur Ausführungsform gemäß Abbildungen 1 und 2 zur Beheizung der Rektifikationskolonne **RD_{A}** <300> effizienter verwertet werden.

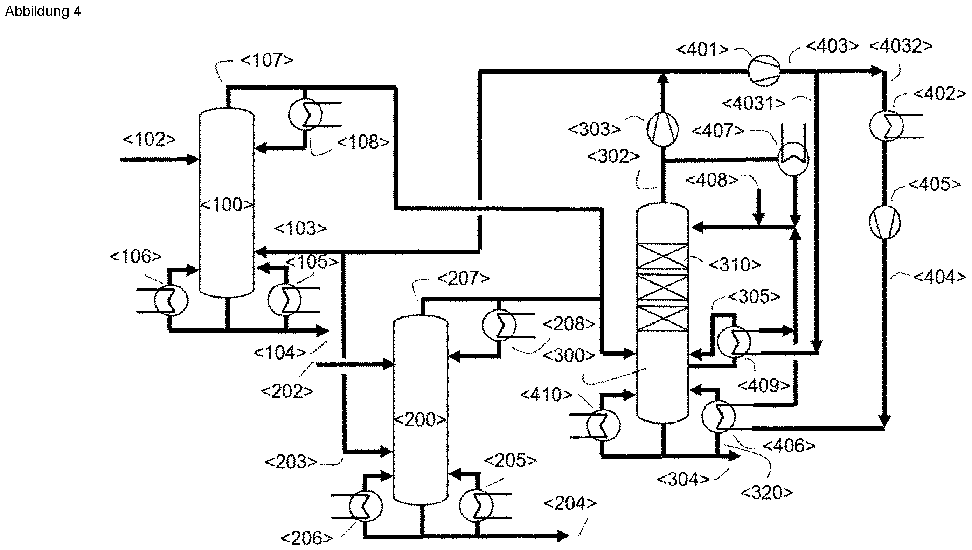

### 3.4 Abbildung 4

Abbildung 4 zeigt eine Ausführungsform des erfindungsgemäßen Verfahrens. Diese entspricht der in Abbildung 3 beschriebenen Ausführungsformen mit dem Unterschied, dass in einer zweiten Reaktionskolonne **RR_{B}** <200> eine wässrige KOH-Lösung **S_{BE2}** <202>, mit Methanol, das auch zur Umsetzung in **RR_{A}** <100> eingesetzt **wird,S_{BE1}** <203> zu dem entsprechenden Kaliummethanolat umgesetzt wird.

Am Kopf der Reaktionskolonne **RR_{B}** <200> wird eine wässrige KOH-Lösung als Eduktstrom **S_{BE2}** <202> zugegeben. Alternativ kann auch eine methanolische KOH-Lösung als Eduktstrom **S_{BE2}** <202> zugegeben werden. Oberhalb des Sumpfes der Reaktionskolonne **RR_{B}** <200> wird Methanol als Eduktstrom **S_{BE1}** <203> dampfförmig zugegeben.

Am Sumpf der Reaktionskolonne **RR_{B}** <200> wird ein Gemisch des entsprechenden Methanolats in Methanol **S_{BP*}** <204> entnommen. Mit dem Sumpfverdampfer **V_{SB}** <205> und dem optionalen Verdampfer **V_{SB'}** <206> am Sumpf der Kolonne **RR_{B}** <200> wird die Konzentration der Kaliummethanolatlösung **S_{BP*}** <204> auf den gewünschten Wert eingestellt.

Am Kopf der Reaktionskolonne **RR_{B}** <200> wird ein Brüdenstrom **S_{BB}** <207> entnommen. Im Kondensator **K_{RRB}** <208> wird ein Teil des Brüdenstroms **S_{BB}** <207> kondensiert und flüssig als Rücklauf auf den Kopf der Reaktionskolonne **RR_{B}** <200> aufgegeben. Kondensator **K_{RRB}** <208> und die Einstellung des Rücklaufs sind jedoch optional.

Der erhaltene Brüden **S_{BB}** <207> wird vermischt mit dem nicht im Kondensator **K_{RRA}** <108> kondensierten Teil des Brüden **S_{AB}** <107> der Rektifikationskolonne **RD_{A}** <300> zugeführt.

Ein weiterer Unterschied zur Ausführungsform gemäß Abbildung 3 ist, dass der vorverdichtete Teil des Brüden **S_{OA}** <302> teilweise zur Reaktionskolonne **RR_{A}** <100> und **RR_{B}** <200> rezykliert wird, wo er als Eduktstrom **S_{AE1}** <103> bzw. **S_{BE1}** <203> eingesetzt wird.

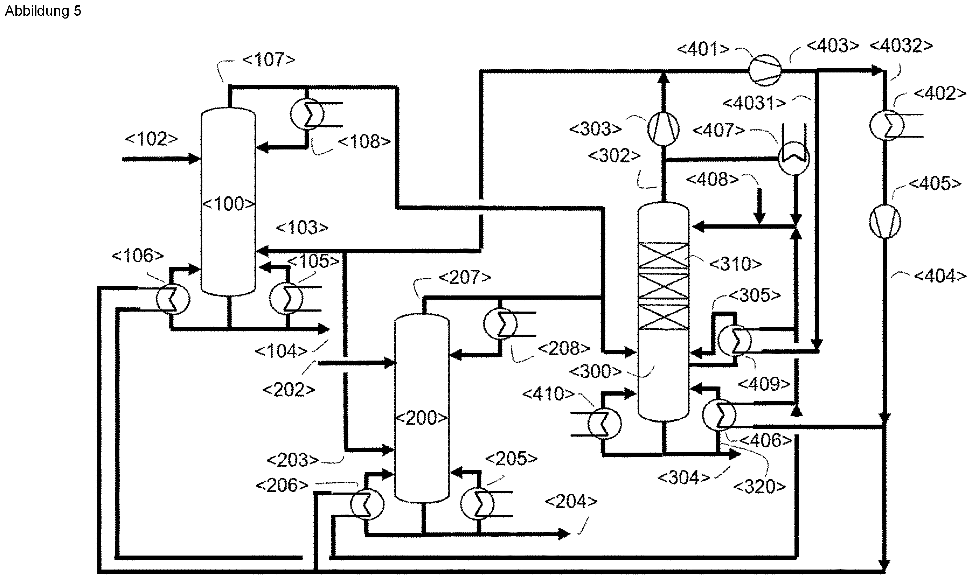

### 3.5 Abbildung 5

Abbildung 5 zeigt eine Ausführungsform des erfindungsgemäßen Verfahrens. Diese entspricht der in Abbildung 4 beschriebenen Ausführungsformen mit dem Unterschied, dass ein Teil von **S_{OA2}** <404> auch zur Beheizung des Verdampfers **V_{SA'}** <106> am Sumpf der Kolonne **RR_{A}** <100> und des Verdampfers **V_{SB'}** <206> am Sumpf der Kolonne **RR_{B}** <200> genutzt wird.

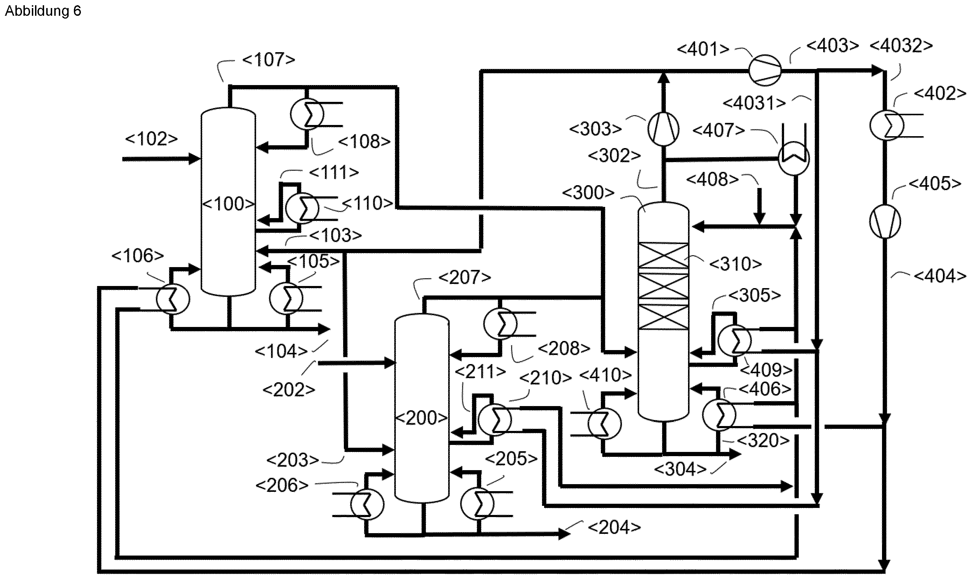

### 3.6 Abbildung 6

Abbildung 6 zeigt eine Ausführungsform des erfindungsgemäßen Verfahrens. Diese entspricht der in Abbildung 5 beschriebenen Ausführungsform mit dem Unterschied, dass die Reaktionskolonnen **RR_{A}** <100> und **RR_{B}** <200> jeweils einen Zwischenverdampfer **V_{ZA}** <110> bzw. **V_{ZB}** <210> aufweisen. Ein Seitenstrom **S_{ZAA}** <111> wird der Reaktionskolonne **RR_{A}** <100> entnommen und über **V_{ZA}** <110> geführt, danach der Reaktionskolonne **RR_{A}** <100> wieder zugeführt. Ein Seitenstrom **S_{ZBA}** <211> wird der Reaktionskolonne **RR_{B}** <200> entnommen und über **V_{ZB}** <210> geführt, danach der Reaktionskolonne **RR_{B}** <200> wieder zugeführt.

Im Unterschied zu Abbildung 5 wird ein Teil von **S_{OA2}** <404> nur zur Beheizung des Verdampfers **V_{SA'}** <106> am Sumpf der Kolonne **RR_{A}** <100> genutzt. Dagegen wird ein Teil von **S_{OA11}** <4031> zur Beheizung des Verdampfers **V_{ZB}** <210> genutzt.

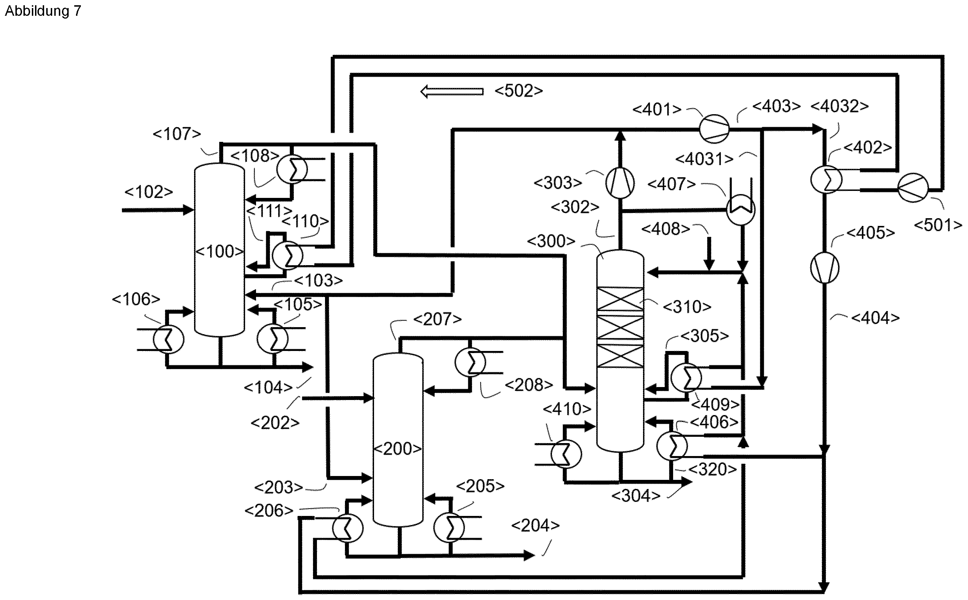

### 3.7 Abbildung 7

Abbildung 7 zeigt eine Ausführungsform des erfindungsgemäßen Verfahrens. Diese entspricht der in Abbildung 5 beschriebenen Ausführungsformen mit dem Unterschied, dass die Reaktionskolonne **RR_{A}** <100> einen Zwischenverdampfer **V_{ZA}** <110> aufweist. Ein Seitenstrom **S_{ZAA}** <111> wird der Reaktionskolonne **RR_{A}** <100> entnommen und über **V_{ZA}** <110> geführt, danach der Reaktionskolonne **RR_{A}** <100> wieder zugeführt. Im Unterschied zu Abbildung 5 wird ein Teil von **S_{OA2}** <404> nur zur Beheizung des Verdampfers **V_{SB'}** <206> am Sumpf der Kolonne **RR_{B}** <200> eingesetzt. Der Zwischenverdampfer **V_{ZA}** <110> wird über ein durch eine Pumpe <501> transportiertes Wärmeträgermedium **W** <502>, insbesondere Wasser, beheizt, welches Wärme im Zwischenkühler **WT_{X}** <402> von **S_{OA12}** <4032> aufnimmt und im Zwischenverdampfer **V_{ZA}** <110> abgibt.

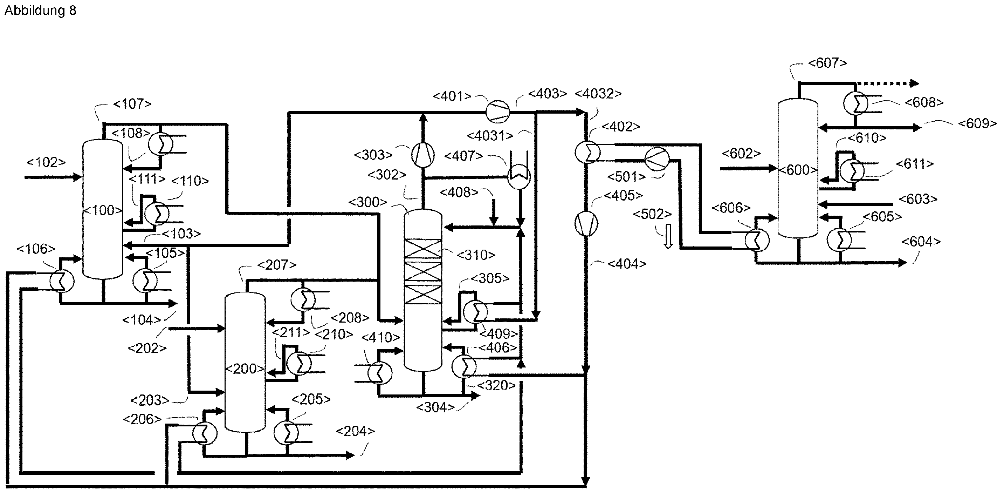

### 3.8 Abbildung 8

Abbildung 8 zeigt eine Ausführungsform des erfindungsgemäßen Verfahrens. Diese entspricht der in Abbildung 5 beschriebenen Ausführungsform mit dem Unterschied, dass die Reaktionskolonnen **RR_{A}** <100> und **RR_{B}** <200> jeweils einen Zwischenverdampfer **V_{ZA}** <110> bzw. **V_{ZB}** <210> aufweisen. Ein Seitenstrom **S_{ZAA}** <111> wird der Reaktionskolonne **RR_{A}** <100> entnommen und über **V_{ZA}** <110> geführt, danach der Reaktionskolonne **RR_{A}** <100> wieder zugeführt. Ein Seitenstrom **S_{ZBA}** <211> wird der Reaktionskolonne **RR_{B}** <200> entnommen und über **V_{ZB}** <210> geführt, danach der Reaktionskolonne **RR_{B}** <200> wieder zugeführt.

Daneben zeigt Abbildung 8 eine weitere bevorzugte Ausführungsform des erfindungsgemäßen Verfahrens. Es zeigt eine Reaktivrektifikationskolonne **RR_{c}** <600> zur Umalkoholisierung von Natriummethanolat zu Natriumethanolat, die mind. teilweise mit Energie aus dem Strom **S_{OA12}** <4032> betrieben wird. Die Kolonne **RR_{c}** <600> weist die Sumpfverdampfer **V_{sc}** <605> und **V_{sc'}** <606> auf.

Dabei wird Natriummethanolatlösung **S_{CE1}** <602> in einer Reaktionskolonne **RR_{C}** <600> im Gegenstrom mit Ethanol **S_{CE2}** <603> zu Natriumethanolat umgesetzt und dieses als ethanolische Lösung entnommen.

Am Sumpf der Reaktionskolonne **RR_{C}** <600> wird ein Sumpfproduktstrom **S_{CP}** <604> umfassend Natriumethanolat entnommen.

Am Kopf der Reaktionskolonne **RR_{C}** <600> wird ein Brüdenstrom **S_{CB}** <607> entnommen. Im Kondensator **K_{RRC}** <608> wird dabei mindestens ein Teil des Brüdenstroms **S_{CB}** <607> kondensiert und davon mindestens ein Teil flüssig als Rücklauf auf den Kopf der Reaktionskolonne **RR_{C}** <600> aufgegeben. Der Brüdenstroms **S_{CB}** <607> wird dabei entweder gasförmig vor dem Kondensator **K_{RRC}** <608> abgezogen (eingezeichnet durch gestrichelte Linie) und/oder als Strom <609> flüssig hinter dem Kondensator **K_{RRC}** <608>.

Ein Seitenstrom **S_{ZC}** <610> wird der Reaktionskolonne **RR_{C}** <600> bevorzugt entnommen, wobei auf diesen über einen Zwischenverdampfer **V_{ZC}** <611> Energie übertragen wird und **S_{ZC}** <610> danach wieder in **RR**_{C} <600> zurückgeleitet werden kann.

Als Natriummethanolatlösung **S**_{CE1} <602> wird bevorzugt mindestens ein Teil der in der Reaktionskolonne **RR**_{A} <100> und **RR**_{B} <200> gewonnenen Sumpfströme **S**_{AP*} <104> bzw. **S**_{BP*} <204> genutzt.

Der Sumpfverdampfer **V**_{SC'} <606> wird über ein durch eine Pumpe <501> transportiertes Wärmeträgermedium **W**₁ <502>, insbesondere Wasser, beheizt, welches Wärme im Zwischenkühler **WT**_{X} <402> von **S**_{OA12} <4032> aufnimmt und im Sumpfverdampfer **V**_{SC'} <606> abgibt.

Alternativ kann auch Energie entsprechend von einem anderen Strom ausgewählt aus **S**_{OA2} <404>, **S**_{OA11} <4031>, **S**_{OA1} <403> vor der Auftrennung in **S**_{OA11} und **S**_{OA12}, auf den Sumpfverdampfer **V**_{SC'} <606> oder den anderen Sumpfverdampfer **V**_{SC} <605> übertragen werden. Von mindestens einem der Ströme **S**_{OA1} <403>, **S**_{OA11} <4031>, **S**_{OA12} <4032>, **S**_{OA2} <404> kann ebenso Energie auf den Ethanolstrom **S**_{CE1} <603>, die Natriummethanolatlösung **S**_{CE1} <602> oder den Seitenstrom **S**_{ZC} <610> übertragen werden.

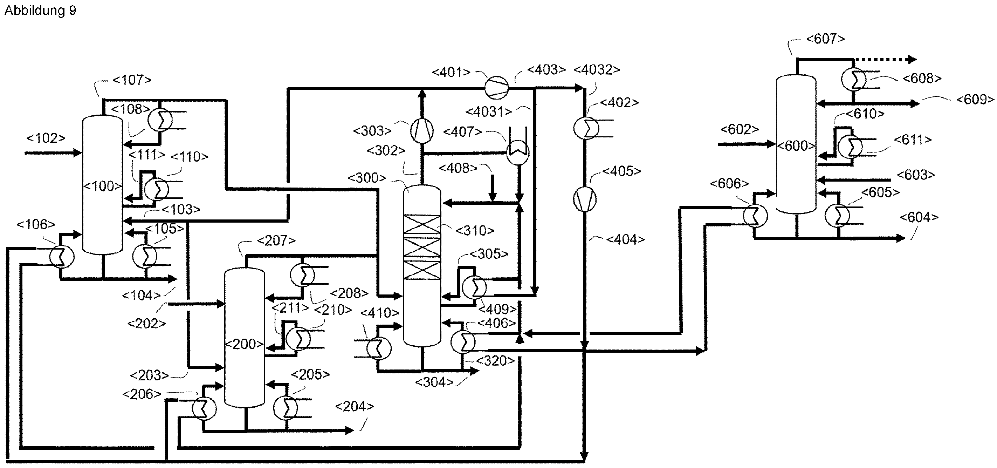

### 3.9 Abbildung 9

Abbildung 9 zeigt eine Ausführungsform des erfindungsgemäßen Verfahrens. Diese entspricht der in Abbildung 8 beschriebenen Ausführungsform mit dem Unterschied, dass die Beheizung des Sumpfverdampfers **V**_{sc'} <606> direkt mit einem Teil von **S**_{OA2} <404> erfolgt.

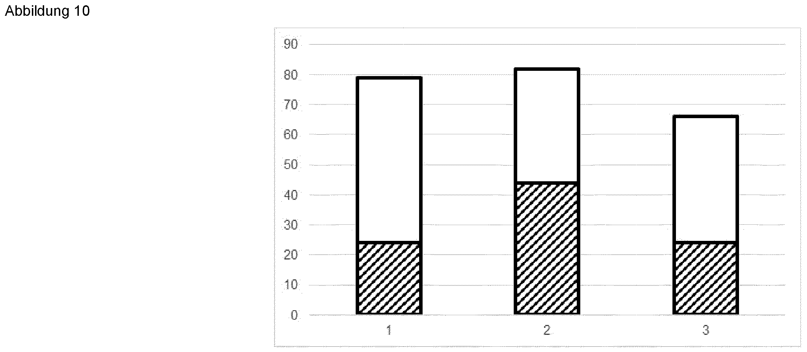

### 3.10 Abbildung 10

Abbildung 10 illustriert die Energieersparnis im erfindungsgemäßen Verfahren nach Beispiel 3 gegenüber dem nicht erfindungsgemäßen Verfahren gemäß Beispiel 1 und 2. Die x-Achse bezeichnet das jeweilige Beispiel, die y-Achse die aufzubringende Leistung in kW (Heizdampf- und Verdichterleistung).

Der schraffierte Teil der Balken gibt die nötige Heizleistung durch Niederdruckdampf wieder, der weiße Teil der Balken die Summe der Verdichterleistungen.

### 4. Detaillierte Beschreibung der Erfindung

Die vorliegende Erfindung betrifft ein Verfahren zur Herstellung mindestens eines Alkalimetallalkoholats der Formel M_{A}OR, wobei R Methyl ist, und wobei M_{A} ein Metall ausgewählt aus Natrium, Kalium, bevorzugt Natrium, ist.

Das erfindungsgemäße Verfahren umfasst mit den Schritten (a) bis (f) ein Verfahren zur Aufarbeitung eines Gemisches **G** umfassend Wasser und Alkohol ROH, wobei R Methyl ist.

ROH ist demnach Methanol.

### 4.1 Verfahren zur Aufarbeitung eines Gemisches G

Das erfindungsgemäße Verfahren zur Aufarbeitung eines Gemisches **G** umfasst die Schritte (a) bis (f) des erfindungsgemäßen Verfahrens zur Herstellung mindestens eines Alkalimetallalkoholats der Formel M_{A}OR.

Das Gemisch **G** ist insbesondere gasförmig, dann wird es auch als "Brüden" bezeichnet.

Bei dem Gemisch **G** handelt es sich um den Kopfstrom einer Reaktionskolonne, in der der Alkohol ROH mit einem Alkalimetallhydroxid NaOH oder KOH zum entsprechenden Alkalimetallalkoholat NaOR bzw. KOR umgesetzt wurde.

Erfindungsgemäß wird als das in Schritt (a) eingesetzte Gemisch **G** mindestens ein Teil des Brüdenstroms **S_{AB},** und, wenn Schritt (α2) durchgeführt wird, mindestens ein Teil des Brüdenstrom **S_{BB},** eingesetzt.

Der Brüdenstroms **S_{AB}** wird in Schritt (α1) erhalten (beschrieben in Punkt 4.2.1).

Der Brüdenstrom **S_{BB}** wird im optionalen Schritt (α2) erhalten, wenn dieser Schritt durchgeführt wird (beschrieben in Punkt 4.2.2).

Der Brüdenstrom **S_{BB}** wird nur dann erhalten, wenn der optionale Schritt (α2) durchgeführt wird. Wenn der optionale Schritt (α2) durchgeführt wird, **S_{BB}** erhalten wird und mindestens ein Teil von **S_{BB}** als Gemisch **G** eingesetzt wird, wird der mindestens eine Teil von **S_{BB}** vermischt mit **S_{AB}** oder getrennt von **S_{AB}** als Gemisch **G** eingesetzt.

Im Schritt (β) des Verfahrens wird mindestens ein Teil des Brüdenstroms **S_{AB},** und, wenn Schritt (α2) durchgeführt wird, mindestens ein Teil des Brüdenstrom **S_{BB},** vermischt mit **S_{AB}** oder getrennt von **S_{AB},** als Gemisch **G** in Schritt (a) eines Verfahrens zur Aufarbeitung eines Gemisches **G** umfassend Wasser und Alkohol ROH eingesetzt.

Das Gemisch **G** umfassend Wasser und Alkohol ROH wird erfindungsgemäß mit dem Verfahren zur Aufarbeitung eines Gemisches **G** aufgearbeitet.

### 4.1.1 Schritt (a) des erfindungsgemäßen Verfahrens

In Schritt (a) des erfindungsgemäßen Verfahrens wird das Gemisch **G** in eine Rektifikationskolonne **RD_{A}** geleitet und in **RD_{A}** in mindestens einen Brüdenstrom **S_{OA}** umfassend ROH, der am oberen Ende von **RD_{A}** entnommen wird, und mindestens einen Strom **S_{UA}** umfassend Wasser, der am unteren Ende von **RD_{A}** entnommen wird, aufgetrennt.

"Mindestens ein Brüdenstrom **S_{OA}** umfassend ROH, der am oberen Ende von **RD_{A}** entnommen wird" bedeutet, dass der Brüden, der am oberen Ende von **RD_{A}** erhalten wird, dort als ein oder mehrere Brüdenströme entnommen werden kann. Falls er dort in mehr als einem Brüdenstrom entnommen wird, werden die m Brüdenströme als "Brüdenstrom **S_{OAI}",** "Brüdenstrom **S_{OAII}",** [...], "Brüdenstrom **S_{OAm}"** bezeichnet, wobei "m" die Anzahl der am oberen Ende von **RD_{A}** entnommenen Brüdenströme (in römischen Zahlen) angibt.

"Mindestens einen Strom **S_{UA}** umfassend Wasser, der am unteren Ende von **RD_{A}** entnommen wird" bedeutet, dass Wasser, das am unteren Ende von **RD_{A}** erhalten wird, dort als ein oder mehrere Ströme entnommen werden kann. Falls es dort in mehr als einem Strom entnommen wird, werden die n Ströme als "Strom **S_{UAI}",** "Strom **S_{UAII}",** [...], "Strom **S_{UAn}"** bezeichnet, wobei "n" die Anzahl der am unteren Ende von **RD_{A}** entnommenen Ströme (in römischen Zahlen) angibt.

Das Gemisch **G** kann dabei über eine oder mehrere Zulaufstellen in die Rektifikationskolonne **RD_{A}** geleitet werden. Über mehrere Zulaufstellen wird es beispielsweise in den Ausführungsformen geleitet, in denen im Verfahren gemäß dem bevorzugten Aspekt der Erfindung Schritt (α2) durchgeführt wird und in Schritt (β) mindestens ein Teil des Brüdenstroms **S_{BB}** getrennt von **S_{AB},** als Gemisch **G** in Schritt (a) des Verfahrens eingesetzt wird. In dieser Ausführungsform wird demnach das Gemisch **G** als zwei voneinander getrennte Ströme in die Rektifikationskolonne **RD_{A}** geleitet.

In den Ausführungsformen der vorliegenden Erfindung, in denen das Gemisch **G** als zwei oder mehrere voneinander getrennte Ströme in die Rektifikationskolonne **R_{DA}** geleitet wird, ist es vorteilhaft, wenn die Zulaufstellen der einzelnen Ströme im Wesentlichen auf der gleichen Höhe an der Rektifikationskolonne **RD_{A}** liegen.

In einer bevorzugten Ausführungsform des Schritts (a) des erfindungsgemäßen Verfahrens wird das Gemisch **G** in einer Rektifikationskolonne **RD_{A}** in einen Brüdenstrom **S_{OA}** umfassend ROH, der am oberen Ende von **RD_{A}** entnommen wird, und einen Strom **S_{UA}** umfassend Wasser, der am unteren Ende von **RD_{A}** entnommen wird, aufgetrennt.

Eine andere Bezeichnung für "oberes Ende einer Rektifikationskolonne" ist "Kopf".

Eine andere Bezeichnung für "unteres Ende einer Rektifikationskolonne" ist "Sumpf" oder "Fuß".

Der Druck, den der mindestens eine Brüdenstrom **S**_{OA} aufweist, wird mit "**p**_{OA}", seine Temperatur mit "**T**_{OA}" bezeichnet. Dies bezieht sich insbesondere auf Druck und Temperatur des mindestens einen Brüdenstroms **S**_{OA}, wenn er in Schritt (a) der Rektifikationskolonne **RD_{A}** entnommen wird.

Der Druck **p**_{OA} liegt insbesondere im Bereich von 0.5 bar abs. bis 8 bar abs., bevorzugter im Bereich von 0.6 bar abs. bis 7 bar abs., bevorzugter im Bereich von 0.7 bar abs. bis 6 bar abs., noch bevorzugter im Bereich von 1 bar abs. bis 5 bar abs., und ist am bevorzugtesten 1 bar abs. bis 4 bar abs.

Die Temperatur **T**_{OA} liegt insbesondere im Bereich von 45 °C bis 150 °C, bevorzugter im Bereich von 48 °C bis 140 °C, bevorzugter im Bereich von 50 °C bis 130 °C, noch bevorzugter im Bereich von 60 °C bis 120 °C, und ist am bevorzugtesten im Bereich von 60 °C bis 110 °C.

Als Rektifikationskolonne **RD_{A}** in Schritt (a) des Verfahrens kann jede beliebige, dem Fachmann bekannte Rektifikationskolonne eingesetzt werden. Bevorzugt enthält die Rektifikationskolonne **RD_{A}** Einbauten. Geeignete Einbauten sind zum Beispiel Böden, unstrukturierte Packungen oder strukturierte Packungen. Als Böden werden üblicherweise Glockenböden, Siebböden, Ventilböden, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel oder Intalox^{®}-Sättel verwendet. Strukturierte Packungen werden zum Beispiel unter dem Handelsnamen Mellapack^{®} der Firma Sulzer vertrieben. Neben den genannten Einbauten weitere geeignete Einbauten sind dem Fachmann bekannt und können ebenfalls verwendet werden.

Bevorzugte Einbauten weisen einen geringen spezifischen Druckverlust pro theoretischer Trennstufe auf. Strukturierte Packungen und Füllkörper haben zum Beispiel einen deutlich kleineren Druckverlust pro theoretischer Trennstufe als Böden. Dies hat den Vorteil, dass der Druckverlust in der Rektifikationskolonne **RD_{A}** möglichst gering bleibt und somit die mechanische Leistung des Verdichters und die Temperatur des zu verdampfenden Alkohol/Wasser-Gemischs gering bleibt.

Wenn in der Rektifikationskolonne **RD_{A}** strukturierte Packungen oder unstrukturierte Packungen enthalten sind, so können diese geteilt sein oder es kann eine durchgehende Packung vorliegen. Üblicherweise sind jedoch mindestens zwei Packungen vorgesehen, eine Packung oberhalb der Zulaufstelle des Gemischs **G** und eine Packung unterhalb der Zulaufstelle des Gemisches **G.** Es kann auch eine Packung oberhalb der Zulaufstelle des Gemischs **G** und mehrere Böden unterhalb der Zulaufstelle des Gemisches **G** vorgesehen sein. Wenn eine unstrukturierte Packung eingesetzt wird, beispielsweise eine Füllkörperpackung, so liegen die Füllkörper üblicherweise auf einem geeigneten Tragrost (z.B. Siebboden oder Gitterboden) auf.

Im Schritt (a) des Verfahrens gemäß der Erfindung wird dann der mindestens eine Brüdenstrom **S_{OA}** umfassend ROH am oberen Ende der Rektifikationskolonne **RD_{A}** entnommen. Der bevorzugte Massenanteil von ROH in diesem Brüdenstrom **S_{OA}** ist ≥ 99 Gew.-%, bevorzugter ≥ 99.6 Gew.-%, noch bevorzugter ≥ 99.9 Gew.-%, wobei der Rest insbesondere Wasser ist.

Am unteren Ende von **RD_{A}** wird mindestens ein Strom **S_{UA}** umfassend Wasser entnommen, der bevorzugt < 1 Gew.-%, bevorzugter ≤ 5000 Gew.-ppm, noch bevorzugter ≤ 2000 Gew.-ppm Alkohol aufweisen kann.

Die Entnahme des mindestens einen Brüdenstroms **S_{OA}** umfassend ROH am Kopf der Rektifikationskolonne **RD_{A}** bedeutet im Rahmen der vorliegenden Erfindung insbesondere, dass der mindestens eine Brüdenstrom **S_{OA}** als Kopfstrom oder als Seitenabzug oberhalb der Einbauten in der Rektifikationskolonne **RD_{A}** entnommen wird.

Die Entnahme des mindestens einen Stroms **S**_{UA} umfassend Wasser am Sumpf der Rektifikationskolonne **RD**_{A} bedeutet im Rahmen der vorliegenden Erfindung insbesondere, dass der mindestens ein Strom **S**_{UA} als Sumpfstrom oder am unteren Boden der Rektifikationskolonne **RD**_{A} entnommen wird.

Die Rektifikationskolonne **RD**_{A} wird mit oder ohne, vorzugsweise mit Rücklauf betrieben.

"Mit Rücklauf" bedeutet, dass der am oberen Ende der Rektifikationskolonne **RD_{A}** entnommene Brüdenstrom **S**_{OA} nicht vollständig abgeführt wird, sondern teilweise kondensiert und wieder der jeweiligen Rektifikationskolonne **RD**_{A} zugeführt wird. In den Fällen, in denen ein solcher Rücklauf eingestellt wird, beträgt das Rücklaufverhältnis dabei bevorzugt 0.0001 bis 1, bevorzugter 0.0005 bis 0.9, noch bevorzugter 0.001 bis 0.8.

Ein Rücklauf kann dadurch eingestellt werden, dass am Kopf der Rektifikationskolonne **RD_{A}** ein Kondensator **K_{RD}** angebracht wird. In dem Kondensator **K_{RD}** wird der jeweilige Brüdenstrom **S**_{OA} teilweise kondensiert und der Rektifikationskolonne **RD_{A}** wieder zugeführt. Unter einem Rücklaufverhältnis wird allgemein und im Sinne dieser Erfindung das Verhältnis aus dem Anteil des aus der Kolonne entnommenen Massenstroms (kg/h), der wieder auf die Kolonne in flüssiger Form zurückgeführt wird (Rücklauf) zu dem Anteil dieses Massenstroms (kg/h), der in flüssiger Form oder gasförmiger Form von der jeweiligen Kolonne abgeführt wird, verstanden.

### 4.1.2 Schritt (b) des erfindungsgemäßen Verfahrens

In Schritt (b) des erfindungsgemäßen Verfahrens wird mindestens ein Seitenstrom **S_{ZA}** aus **RD_{A}** entnommen und wieder in **RD_{A}** rückgeführt.

In einer bevorzugten Ausführungsform des Schritts (b) des erfindungsgemäßen Verfahrens wird ein Seitenstrom **S_{ZA}** aus **RD_{A}** entnommen und wieder in **RD**_{A} rückgeführt.

"Seitenstrom **S_{ZA}** aus **RD_{A}"** bedeutet erfindungsgemäß, dass der Strom an einer Entnahmestelle **E_{ZA}** unterhalb des Kopfes und oberhalb des Sumpfes von **RD**_{A} entnommen wird und insbesondere zusätzlich an einer Zulaufstelle **Z_{ZA}** (das ist die Stelle, an der der jeweilige Seitenstrom S_{ZA} wieder in die Rektifikationskolonne **RD_{A}** rückgeführt wird) unterhalb des Kopfes und oberhalb des Sumpfes von **RD_{A}** wieder in **RD_{A}** rückgeführt wird.

Dies bedeutet insbesondere, dass die Entnahmestelle **E_{ZA},** und bevorzugt auch die Zulaufstelle **Z_{ZA}** des jeweiligen Seitenstroms **S_{ZA}** an der Rektifikationskolonne **RD_{A}** unterhalb der Entnahmestellen **E_{OA}** aller aus **RD_{A}** entnommenen Brüdenströme **S_{OA}** liegt, bevorzugt mindestens 1, noch bevorzugter mindestens 5, noch bevorzugter mindestens 10 theoretische Stufen unterhalb der Entnahmestelle **E_{OA}** jenes aus **RD_{A}** entnommenen Brüdenstroms **S_{OA},** dessen Entnahmestelle **E_{OA}** am weitesten unten an der Rektifikationskolonne **RD_{A}** liegt, liegt.

Dies bedeutet insbesondere außerdem, dass die Entnahmestelle **E_{ZA},** und bevorzugt auch die Zulaufstelle **Z_{ZA}** des jeweiligen Seitenstroms **S_{ZA}** an der Rektifikationskolonne **RD_{A}** oberhalb der Entnahmestellen **E_{UA}** aller aus **RD_{A}** entnommenen Ströme **S_{UA}** liegt, bevorzugt mindestens 1, noch bevorzugter mindestens 2, noch bevorzugter mindestens 4 theoretische Stufen oberhalb der Entnahmestelle **E_{UA}** des Stroms **S_{UA},** dessen Entnahmestelle **E_{UA}** am weitesten oben an der Rektifikationskolonne **RD_{A}** liegt, liegt.

In den Fällen, in denen mindestens ein Brüdenstrom **S_{OA}** mindestens teilweise wieder in die Rektifikationskolonne **RD_{A}** rückgeführt wird (was der Fall ist, wenn beispielsweise ein Rücklauf an der Rektifikationskolonne **RD_{A}** eingestellt wird), liegt insbesondere außerdem die Zulaufstelle **Z_{OA}** (das ist die Stelle, an der der mindestens eine Brüdenstrom **S_{OA}** mindestens teilweise wieder in die Rektifikationskolonne **RD_{A}** rückgeführt wird) des mindestens einen Brüdenstroms **S_{OA}** oberhalb der Entnahmestellen **E_{ZA}** und insbesondere auch oberhalb der Zulaufstellen **Z_{ZA}** aller aus **RD_{A}** entnommenen Seitenströme **S_{ZA},** bevorzugt mindestens 1, noch bevorzugter mindestens 5, noch bevorzugter mindestens 10 theoretische Stufen oberhalb der höchsten Stelle aller Entnahme- und Zulaufstellen aller aus **RD_{A}** entnommenen Seitenströme **S_{ZA}.**

In den Fällen, in denen mindestens ein Strom **S_{UA}** mindestens teilweise wieder in die Rektifikationskolonne **RD_{A}** rückgeführt wird, liegt insbesondere außerdem die Zulaufstelle **Z_{UA}** (das ist die Stelle, an der der mindestens eine Strom **S_{UA}** mindestens teilweise wieder in die Rektifikationskolonne **RD_{A}** rückgeführt wird) des mindestens einen Stroms **S_{UA}** unterhalb der Entnahmestellen **E_{ZA}** und insbesondere auch unterhalb der Zulaufstellen **Z_{ZA}** aller aus **RD_{A}** entnommenen Seitenströme **S_{ZA},** bevorzugt mindestens 1, noch bevorzugter mindestens 2, noch bevorzugter mindestens 4 theoretische Stufen unterhalb der niedrigsten Stelle aller Entnahme- und Zulaufstellen aller aus **RD_{A}** entnommenen Seitenströme **S_{ZA}.**

Die Entnahmestelle **E_{ZA}** des Seitenstrom **S_{ZA}** und die Zulaufstelle **Z_{ZA}** des Seitenstroms **S_{ZA}** an der Rektifikationskolonne **RD_{A}** können zwischen den gleichen Böden von **RD_{A}** positioniert sein. Es ist jedoch auch möglich, dass sie auf unterschiedlicher Höhe liegen.

In einer bevorzugten Ausführungsform des Verfahrens gemäß der Erfindung liegt die Entnahmestelle **E_{ZA}** und bevorzugt auch die Zulaufstelle **Z_{ZA}** des mindestens einen Seitenstroms **S_{ZA}** an der Rektifikationskolonne **RD_{A}** unterhalb der Zulaufstelle **Z_{G},** an der das Gemisch **G** in die Rektifikationskolonne **RD_{A}** geleitet wird, und oberhalb des Sumpfes von **RD_{A}.** Noch bevorzugter liegen die Entnahmestelle **E_{ZA}** und bevorzugt auch die Zulaufstelle **Z_{ZA}** des mindestens einen Seitenstroms **S_{ZA}** an der Rektifikationskolonne **RD_{A}** auch unterhalb des Verstärkerteils von **RD_{A}.**

In einer besonders bevorzugten Ausführungsform des Verfahrens gemäß der Erfindung liegt die Entnahmestelle **E_{ZA}** und bevorzugter auch die Zulaufstelle **Z_{ZA}** des mindestens einen Seitenstroms **S_{ZA}** an der Rektifikationskolonne **RD_{A}** im oberen 4/5, bevorzugt oberen ¾, bevorzugt oberen 7/10, bevorzugter oberen 2/3, bevorzugter oberen 1/2 des Bereichs an der Rektifikationskolonne **RD_{A},** der unterhalb der Zulaufstelle **Z_{G},** an der das Gemisch **G** in die Rektifikationskolonne **RD_{A}** geleitet wird, liegt, und oberhalb der obersten aller Entnahme- und Zulaufstellen aller aus **RD_{A}** entnommenen Ströme **S_{UA}** liegt.

Noch bevorzugter liegen die Entnahmestelle **E_{ZA}** und bevorzugt auch die Zulaufstelle **Z_{ZA}** des mindestens einen Seitenstroms **S_{ZA}** an der Rektifikationskolonne **RD_{A}** dann auch unterhalb des Verstärkerteils von **RD_{A}.**

In einer weiteren besonders bevorzugten Ausführungsform des Verfahrens gemäß der Erfindung enthält die Rektifikationskolonne **RD_{A}** einen Verstärkerteil, und es liegt die Entnahmestelle **E_{ZA}** und bevorzugter auch die Zulaufstelle **Z_{ZA}** des mindestens einen Seitenstroms **S_{ZA}** an der Rektifikationskolonne **RD_{A}** im oberen 4/5, bevorzugt oberen ¾, bevorzugt oberen 7/10, bevorzugter oberen 2/3, bevorzugter oberen 1/2 des Bereichs an der Rektifikationskolonne **RD_{A},** der unterhalb des Verstärkerteils und oberhalb der obersten aller Entnahme- und Zulaufstellen aller aus **RD_{A}** entnommenen Ströme **S_{UA}** liegt.

### 4.1.3 Schritt (c) des erfindungsgemäßen Verfahrens

Im Schritt (c) des erfindungsgemäßen Verfahrens wird mindestens ein Teil des mindestens einen Brüdenstroms **S_{OA}** ("mindestens ein Teil des mindestens einen Brüdenstroms **S_{OA}"** = "mindestens ein Teil von **S_{OA}**") verdichtet. Dadurch wird ein gegenüber **S_{OA}** verdichteter Brüdenstrom **S_{OA1}** erhalten.

Der Druck, den der Brüdenstrom **S**_{OA1} aufweist, wird mit "**p**_{OA1}", seine Temperatur mit "**T**_{OA1}" bezeichnet.

Der Druck **p**_{OA1} ist höher als **p**_{OA}. Der genaue Wert von **p**_{OA1} kann in Abhängigkeit der Anforderungen in Schritt (d) vom Fachmann eingestellt werden, solange die Bedingung **p**_{OA1} > **p**_{OA} erfüllt ist. Der Quotient aus **p**_{OA1}/**p**_{OA} (Drücke jeweils in bar abs.) liegt bevorzugt im Bereich von 1.1 bis 10, bevorzugter 1.2 bis 8, bevorzugter 1.25 bis 7, am bevorzugtesten 1.3 bis 6.

Die Temperatur **T**_{OA1} ist insbesondere höher als die Temperatur **T**_{OA}, und der Quotient aus **T**_{OA1}/**T**_{OA} (Temperatur jeweils in °C) liegt bevorzugt im Bereich von 1.03 bis 10, bevorzugter 1.04 bis 9, bevorzugter 1.05 bis 8, bevorzugter 1.06 bis 7, bevorzugter 1.07 bis 6, am bevorzugtesten 1.08 bis 5.

Die bevorzugten Werte von **p_{OA1}** und **T_{OA1}** gelten auch bevorzugt für **S_{OA11}** und **S_{OA12}.**

Das Verdichten des mindestens einen Teils des Brüdenstroms **S_{OA}** in Schritt (c) kann auf jede beliebige, dem Fachmann bekannte Art erfolgen. So kann die Verdichtung zum Beispiel mechanisch und einstufig oder mehrstufig, bevorzugt mehrstufig, durchgeführt werden. Bei einer mehrstufigen Verdichtung können mehrere Verdichter gleicher Bauart oder Verdichter unterschiedlicher Bauart eingesetzt werden. Eine mehrstufige Verdichtung kann mit einer oder mehreren Verdichtermaschinen erfolgen. Der Einsatz einer einstufigen Verdichtung oder einer mehrstufigen Verdichtung ist abhängig vom Verdichtungsverhältnis und somit davon, auf welchen Druck der Brüden **S_{OA}** verdichtet werden soll.

Als Verdichter im erfindungsgemäßen Verfahren, insbesondere zum Verdichten der Brüdenströme **S_{OA}** zu **S_{OA1}** oder **S_{OA12}** zu **S_{OA2},** eignet sich jeder beliebige, dem Fachmann bekannte Verdichter, bevorzugt mechanische Verdichter, mit dem sich Gasströme verdichten lassen. Geeignete Verdichter sind zum Beispiel ein- oder mehrstufige Turbinen, Kolbenverdichter, Schraubenverdichter, Zentrifugalverdichter oder Axialverdichter.

Bei einer mehrstufigen Verdichtung werden für die jeweils zu überwindenden Druckstufen geeignete Verdichter eingesetzt.

### 4.1.4 Schritt (d) des erfindungsgemäßen Verfahrens

Im Schritt (d) des Verfahrens gemäß der Erfindung wird Energie von einem ersten Teil **S_{OA11}** des verdichteten Brüdenstroms **S_{OA1}** auf **S_{ZA}** übertragen, bevor **S_{ZA}** in **RD_{A}** rückgeführt wird.

**S**_{OA1} wird insbesondere in Schritt (d) zunächst in mindestens zwei Teile **S**_{OA11} und **S**_{OA12} aufgeteilt. Das Verhältnis der Massenströme (in kg/h) von **S**_{OA11} zu **S**_{OA12} liegt bevorzugt im Bereich von 1 : 99 bis 99 : 1, bevorzugter im Bereich von 1 : 50 bis 50 : 1, noch bevorzugter im Bereich von 1: 20 bis 30 : 1, noch bevorzugter im Bereich von 5 : 20 bis 15 : 1.

Im Schritt (d) des erfindungsgemäßen Verfahrens wird Energie vom ersten Teil **S**_{OA11} auf **S**_{ZA} übertragen. Durch den Schritt (d) sinkt die Energie von **S**_{OA11}, so dass insbesondere der Strom **S**_{OA11} mindestens teilweise kondensiert.

"Übertragung von Energie" bedeutet erfindungsgemäß insbesondere "Beheizung", also Übertragung von Energie in Form von Wärme.

"Übertragung von Energie von einem ersten Teil S_{OA11} des verdichteten Brüdenstroms **S**_{OA1} auf **S_{ZA}"** umfasst auch die Fälle, in denen ein Teil von **S_{OA11}** abgetrennt wird und nur von diesem Teil Energie auf **S_{ZA}** übertragen wird. Dies ist beispielsweise in jenen Ausführungsformen der Erfindung der Fall, in denen zusätzlich Energie von **S_{OA11}** auf das Rohprodukt **RP_{A}** und, falls Schritt (α2) durchgeführt wird, alternativ oder zusätzlich auf das Rohprodukt **RP_{B}** übertragen wird (beschrieben in Abschnitt 4.2).

Die Übertragung von Energie von **S_{OA11}** auf **S_{ZA},** bevorzugt die Beheizung von **S_{ZA}** durch **S_{OA11}** erfolgt bevorzugt direkt oder indirekt.

"Direkt" bedeutet, dass **S_{OA11}** mit **S_{ZA}** kontaktiert wird, ohne dass die beiden Ströme sich mischen, so dass Energie, insbesondere Wärme, von **S_{OA11}** auf **S_{ZA}** übergeht.

Dies kann durchgeführt werden, in dem **S_{OA11}** und **S_{ZA}** durch einen Zwischenverdampfer **V_{ZRD}** an der Rektifikationskolonne **RD_{A}** geleitet werden und **S_{OA11}** den **S_{ZA}** beheizt.

Als Wärmeüberträger (anderer Begriff für "Wärmeüberträger" = "Wärmetauscher"), insbesondere als die nachfolgend genannten Wärmeüberträger **WT**_{X}, **WT**_{Y}, **WT**_{Z}, können die dem Fachmann geläufigen Wärmeüberträger, insbesondere Verdampfer, eingesetzt werden. In Schritt (d) des Verfahrens gemäß der Erfindung erfolgt insbesondere die Übertragung der Energie, bevorzugter Wärme, von **S**_{OA11} auf **S**_{ZA} in einem Zwischenverdampfer **V**_{ZRD}.

"Indirekt" bedeutet insbesondere, dass **S**_{OA11} mit einem Wärmeträger **W**₁, bevorzugt über mindestens einen Wärmeüberträger **WT**_{X}, kontaktiert wird, wobei es sich bei dem Wärmeträger nicht um **S_{ZA}** handelt, **W**₁ also von **S_{ZA}** verschieden ist, so dass Energie, bevorzugt Wärme, von **S**_{OA11} auf **W₁** übergeht, ohne dass die beiden Ströme sich mischen, und die Wärme dann von **W**₁ auf **S_{ZA}** übergeht, in dem **W₁ S_{ZA}** kontaktiert, wobei sich **S_{ZA}** und **W₁** mischen oder nicht mischen, bevorzugt aber nicht mischen. Wenn sich **W₁** und **S_{ZA}** dabei nicht mischen, erfolgt die Übertragung der Energie, bevorzugt Wärme, insbesondere in einem weiteren Wärmeüberträger **WT_{Y}.**

In einer weiteren Ausführungsform des erfindungsgemäßen Verfahrens kann bei indirekter Energieübertragung von **S_{OA11}** auf **S_{ZA},** insbesondere Beheizung von **S_{ZA}** durch **S_{OA11},** auch zunächst Energie, bevorzugt Wärme von **S_{OA11} auf W₁,** bevorzugt durch Kontaktierung über mindestens einen Wärmetauscher **WT_{X}** übertragen werden, und dann von **W₁** auf einen weiteren, von **S_{ZA}** verschiedenen Wärmeträger **W₂,** bevorzugt durch Kontaktierung über mindestens einen Wärmetauscher **WT_{Y},** übertragen werden. Im letzten Schritt erfolgt dann Übertragung der Wärme von **W₂** auf **S_{ZA},** wobei sich **S_{ZA}** und **W₂** mischen oder nicht mischen, bevorzugt aber nicht mischen. Wenn sich **W₂** und **S_{ZA}** dabei nicht mischen, erfolgt die Übertragung der Energie, bevorzugt Wärme, insbesondere in einem weiteren Wärmeüberträger **WT_{Z}.**

Es versteht sich von selbst, dass sich in weiteren Ausführungsformen der vorliegenden Erfindung dementsprechend noch weitere Wärmeträger **W₃, W₄, W₅** etc. nutzen lassen.

Als Wärmeträger **W₁** bzw. daneben weiter genutzte Wärmeträger **W₂, W₃, W₄, W₅** kann jeder dem Fachmann bekannte Wärmeträger genutzt werden, bevorzugt sind sie ausgewählt aus der Gruppe bestehend aus Wasser; Alkohol-Wasser-Lösungen; Salz-Wasser-Lösungen, wozu auch ionische Flüssigkeiten, wie zum Beispiel LiBr-Lösungen, Dialkylimidazoliumsalze wie insbesondere Dialkylimidazoliumdialkylphosphate, gehören; Mineralöle, wie zum Beispiel Dieselöle; Thermalöle wie zum Beispiel Silikonöle; biologische Öle wie zum Beispiel Limonen; aromatische Kohlenwasserstoffe wie zum Beispiel Dibenzyltoluol. Am bevorzugtesten ist als Wärmeträger **W₁** Wasser.

Salz-Wasser-Lösungen, die verwendet werden können, sind beispielsweise auch in der DE 10 2005 028 451 A1 und der WO 2006/134015 A1 beschrieben.

Im Anschluss an Schritt (d) kann **S_{OA11}** dann wieder der Rektifikationskolonne **RD_{A},** gegebenenfalls zusammen mit Frischalkohol und/oder mit dem Rücklauf der Rektifikationskolonne **RD_{A},** eingespeist werden. In einer bevorzugten Ausführungsform wird von **S_{OA11},** insbesondere nach der Übertragung der Energie auf **S_{ZA},** weiter Energie übertragen.

In einer bevorzugten Ausführungsform des Verfahrens gemäß der Erfindung, wird Energie, bevorzugt Wärme, von **S_{OA11},** nachdem **S_{OA11}** Energie auf **S_{ZA}** gemäß Schritt (d) übertragen hat, auf **S_{OA}** übertragen, insbesondere auf den Teil von **S_{OA}** übertragen, der einer Verdichtung, bevorzugt der Verdichtung in Schritt (c), zugeführt wird, wobei es sich um eine Vorverdichtung von **S_{OA}** oder die Verdichtung von **S_{OA}** zu **S_{OA1}** handeln kann. Bevorzugt handelt es sich dabei um die erste Verdichtung, der der Strom **S_{OA},** nachdem der die Kolonne **RD_{A}** verlassen hat, unterworfen wird. Dies ermöglicht es, einen Teil der noch von **S_{OA11}** gespeicherten Restenergie bzw. Restwärme im Prozess einzusetzen, in diesem Fall zur Erwärmung von zu verdichtendem **S_{OA}.**

Dadurch werden eventuell in **S_{OA}** vorhandene Tröpfchen verdampft und so Tropfeneintrag in den Verdichter verhindert.

Andere, bevorzugte zusätzliche Senken für die Energie, bevorzugt Wärme in **S_{OA11},** sind weiter unten beschrieben (siehe Absatz 4.3).

Der Schritt (d) des erfindungsgemäßen Verfahrens spiegelt einen Aspekt des unerwarteten Effekts der vorliegenden Erfindung wider. Dabei dissipiert die bei der Verdichtung des Brüdenstrom **S_{OA}** zum verdichteten Brüdenstrom **S_{OA1}** erhaltene überschüssige Energie nicht ungenutzt, sondern wird in der Rektifikation eingesetzt. Dies erfolgt dergestalt, dass zuerst die Verdichtung von **S_{OA} zu S_{OA1}** erfolgt, womit eine Einstellung auf den Wert, der für eine Energieübertragung von **S_{OA11}** auf **S_{ZA}** optimal ist, ermöglicht wird, und dann ein von **S_{OA11}** verschiedener Teil **S_{OA12}** weiter auf **S_{OA2}** verdichtet werden kann. Die Kondensationswärme, die bei der weiteren Verdichtung von **S_{OA12}** weiter auf **S_{OA2}** erhalten wird, wird in die Kolonne im Sumpfverdampfer eingespeist. Die erforderliche zusätzliche Verdichterleistung ist geringer als die dadurch eingesparte Heizdampfleistung. Das erfindungsgemäße Verfahren benötigt weniger Energie als jene des Standes der Technik, wie sie in Beispielen 1 und 2 gezeigt sind. Mit der Verdichtung auf **S_{OA2}** können Druck- und Temperatur von **S_{OA2}** so eingestellt werden, dass eine optimale Energieübertragung von **S_{OA2}** auf **S_{UA1}** bzw. **S_{UA}** erfolgen kann.

### 4.1.5 Schritt (e) des erfindungsgemäßen Verfahrens

In Schritt (e) des Verfahrens der Erfindung wird ein von **S_{OA11}** verschiedener Teil des verdichteten Brüdenstroms **S_{OA1}, S_{OA12},** weiter verdichtet wird, wodurch ein gegenüber **S_{OA11}** verdichteter Brüdenstrom **S**_{OA2} erhalten wird.

Es versteht sich von selbst, dass **S**_{OA2} nach Durchführung des Schrittes (e) auch gegenüber **S**_{OA12} und **S**_{OA1} verdichtet ist.

Der Druck, den der Brüdenstrom **S**_{OA2} aufweist, wird mit "**p**_{OA2}", seine Temperatur mit "**T**_{OA2}" bezeichnet.

Der Druck **p**_{OA2} ist höher als **p**_{OA1}, und der Quotient aus **p**_{OA2}/**p**_{OA1} (Drücke jeweils in bar abs.) liegt bevorzugt im Bereich von 1.1 bis 10, bevorzugter 1.2 bis 8, bevorzugter 1.25 bis 7, am bevorzugtesten 1.3 bis 6.

Die Temperatur **T**_{OA2} ist insbesondere höher als die Temperatur **T**_{OA1} und der Quotient aus **T**_{OA2}/**T**_{OA1} (Temperatur jeweils in °C) liegt bevorzugt im Bereich von 1.03 bis 10, bevorzugter 1.04 bis 9, bevorzugter 1.05 bis 8, bevorzugter 1.06 bis 7, bevorzugter 1.07 bis 6, am bevorzugtesten 1.08 bis 5.

Das Verdichten von **S_{OA12}** in Schritt (e) kann nach dem Fachmann geläufigen Verfahren durchgeführt werden. So kann die Verdichtung zum Beispiel mechanisch einstufig oder mehrstufig, bevorzugt mehrstufig, durchgeführt werden. Bei einer mehrstufigen Verdichtung können mehrere Verdichter gleicher Bauart oder Verdichter unterschiedlicher Bauart eingesetzt werden. Der Einsatz einer einstufigen Verdichtung oder einer mehrstufigen Verdichtung ist davon abhängig, auf welchen Druck der Brüden **S_{OA12}** verdichtet werden soll. Eine im Kontext des Schrittes (c) für **S_{OA}** beschriebene Vorverdichtung kann auch für die Verdichtung von **S_{OA12}** zu **S_{OA2}** durchgeführt werden, insbesondere genügt im Schritt (e) aber die Verdichtung in einer Stufe, d.h. unter Verwendung eines Verdichters **VD_{X}.**

### 4.1.6 Schritt (f) des erfindungsgemäßen Verfahrens

Im Schritt (f) des Verfahrens gemäß der Erfindung wird Energie von mindestens einem Teil von **S_{OA2}** auf mindestens einen Teil **S_{UA1}** des mindestens einen Stromes **S_{UA}** übertragen, bevor **S_{UA1}** in **RD_{A}** rückgeführt wird.

Bevorzugt wird im Schritt (f) des Verfahrens gemäß der Energie von mindestens einem Teil von **S_{OA2}** auf einen Teil **S_{UA1}** des mindestens einen Stromes **S_{UA}** übertragen, bevor **S_{UA1}** in **RD_{A}** rückgeführt wird.

Durch den Schritt (f) sinkt die Energie von **S_{OA2},** so dass insbesondere der Strom **S_{OA2}** mindestens teilweise kondensiert

Schritt (f) des erfindungsgemäßen Verfahrens umfasst folgende bevorzugte Ausführungsformen (f1), (f2), (f3):
(f1) Energie wird von mindestens einem Teil von **S_{OA2}** auf einen Teil **S_{UA1}** des mindestens einen Stromes **S_{UA}** übertragen, und **S_{UA1}** dann in **RD_{A}** rückgeführt;
(f2) Energie wird von mindestens einem Teil von **S_{OA2}** auf einen Teil **S_{UA1*}** des mindestens einen Stromes **S_{UA}** übertragen, und von **S_{UA1*}** wird dann ein Teil **S_{UA1}** in **RD_{A}** rückgeführt;
(f3) Energie wird von mindestens einem Teil von **S_{OA2}** auf den gesamten Strom **S_{UA}** übertragen und dann wird der gesamte Strom **S_{UA}** oder nur ein Teil **S_{UA1}** des Stromes **S_{UA},** bevorzugt nur ein Teil **S_{UA1}** des Stromes **S_{UA},** in **RD_{A}** rückgeführt.

Die Übertragung von Energie von mindestens einem Teil von **S_{OA2}** auf den mindestens einen Teil **S_{UA1}** des mindestens einen Stromes **S_{UA},** bevorzugt die Beheizung des mindestens einen Teils **S_{UA1}** des mindestens einen Stromes **S_{UA}** durch mindestens einen Teil von **S_{OA2},** erfolgt bevorzugt direkt oder indirekt.

"Direkt" bedeutet, dass mindestens ein Teil von **S**_{OA2} mit dem mindestens einen Teil **S**_{UA1} des mindestens einen Stromes **S**_{UA} kontaktiert wird, ohne dass die beiden Ströme sich mischen, so dass Energie, insbesondere Wärme, von mindestens einem Teil **S**_{OA2} auf den mindestens einen Teil **S**_{UA1} des mindestens einen Stromes **S**_{UA} übergeht.

Dies kann dies durchgeführt werden, in dem der mindestens eine Teil von **S_{OA2}** und der mindestens eine Teil **S**_{UA1} des mindestens einen Stromes **S_{UA}** durch einen Sumpfverdampfer **V_{SRD}** an der Rektifikationskolonne **RD_{A}** geleitet werden und **S_{OA11}** den mindestens einen Teil **S_{UA1}** des mindestens einen Stromes **S_{UA}** beheizt.

Als Wärmeüberträger, insbesondere als die nachfolgend genannten Wärmeüberträger **WT_{X}, WT_{Y}, WT_{Z}**, können die dem Fachmann geläufigen Wärmetauscher, insbesondere Verdampfer, eingesetzt werden. In Schritt (f) des Verfahrens gemäß der Erfindung erfolgt insbesondere die Übertragung der Energie, bevorzugter Wärme, vom mindestens einen Teil von **S_{OA2}** auf den mindestens einen Teil **S_{UA1}** des mindestens einen Stromes **S_{UA}** in einem Sumpfverdampfer **V_{SRD}.**

"Indirekt" bedeutet insbesondere, dass der mindestens eine Teil von **S_{OA2}** mit mindestens einem Wärmeträger **W₁,** bevorzugt über mindestens einen Wärmetauscher **WT_{X},** kontaktiert wird, wobei es sich bei dem Wärmeträger nicht um den mindestens einen Teil **S_{UA1}** des mindestens einen Stromes **S_{UA}** handelt, **W₁** also davon verschieden ist, so dass Energie, bevorzugt Wärme, vom mindestens einen Teil von **S_{OA2}** auf den mindestens einen Wärmeträger **W₁** übergeht, ohne dass die beiden Ströme sich mischen, und die Wärme dann von **W₁** auf den mindestens einen Teil **S_{UA1}** des mindestens einen Stromes **S_{UA}** übergeht, in dem **W₁** die betreffende Komponente kontaktiert, wobei sich der mindestens eine Teil **S_{UA1}** des mindestens einen Stromes **S_{UA}** und **W₁** mischen oder nicht mischen, bevorzugt aber nicht mischen.

In einer weiteren Ausführungsform des erfindungsgemäßen Verfahrens kann bei indirekter Energieübertragung vom mindestens einen Teil von **S_{OA2}** auf den mindestens einen Teil **S_{UA1}** des mindestens einen Stromes **S_{UA},** insbesondere Beheizung des mindestens einen Teils **S_{UA1}** des mindestens einen Stromes **S_{UA}** durch den mindestens einen Teil von **S_{OA2},** auch zunächst Energie, bevorzugt Wärme von **S_{OA2}** auf **W₁,** bevorzugt durch Kontaktierung über mindestens einen Wärmetauscher **WT_{X}** übertragen werden, und dann von **W₁** auf einen weiteren, von dem mindestens einen Teil **S_{UA1}** des mindestens einen Stromes **S_{UA}** verschiedenen Wärmeträger **W₂,** bevorzugt durch Kontaktierung über mindestens einen Wärmetauscher **WT_{Y},** übertragen werden. Im letzten Schritt erfolgt dann Übertragung der Wärme von **W₂** auf den mindestens einen Teil **S_{UA1}** des mindestens einen Stromes **S_{UA},** wobei sich der mindestens eine Teil **S_{UA1}** des mindestens einen Stromes **S_{UA}** und **W₂** mischen oder nicht mischen, bevorzugt aber nicht mischen. Es versteht sich von selbst, dass sich in weiteren Ausführungsformen der vorliegenden Erfindung dementsprechend noch weitere Wärmeträger **W₃, W₄, W₅** etc. nutzen lassen.

Als Wärmeträger **W₁** bzw. daneben weiter genutzte Wärmeträger **W₂, W₃, W₄, W₅** kann jeder dem Fachmann bekannte Wärmeträger genutzt werden, bevorzugt sind sie ausgewählt aus der Gruppe bestehend aus Wasser; Alkohol-Wasser-Lösungen; Salz-Wasser-Lösungen, wozu auch ionische Flüssigkeiten, wie zum Beispiel LiBr-Lösungen, Dialkylimidazoliumsalze wie insbesondere Dialkylimidazoliumdialkylphosphate, gehören; Mineralöle, wie zum Beispiel Dieselöle; Thermalöle wie zum Beispiel Silikonöle; biologische Öle wie zum Beispiel Limonen; aromatische Kohlenwasserstoffe wie zum Beispiel Dibenzyltoluol. Am bevorzugtesten ist als Wärmeträger **W₁** Wasser.

Salz-Wasser-Lösungen, die verwendet werden können, sind beispielsweise auch in der DE 10 2005 028 451 A1 und der WO 2006/134015 A1 beschrieben.

Im Anschluss an Schritt (f) kann der mindestens eine Teil von **S_{OA2}** dann wieder der Rektifikationskolonne **RD_{A},** gegebenenfalls zusammen mit Frischalkohol und/oder mit dem Rücklauf der Rektifikationskolonne **RD_{A}** und/oder mit dem nach Durchführung des Schritts (d) erhaltenen Strom **S_{OA11}** eingespeist werden. In einer bevorzugten Ausführungsform wird von mindestens einen Teil von **S_{OA2},** insbesondere nach der Übertragung der Energie auf den mindestens einen Teil **S_{UA1}** von **S_{UA},** weiter Energie übertragen.

In einer bevorzugten Ausführungsform des Verfahrens gemäß der Erfindung, wird Energie, bevorzugter Wärme, vom mindestens einen Teil von **S_{OA2},** nachdem von diesem Energie auf den mindestens einen Teil **S_{UA1}** von **S_{UA}** gemäß Schritt (f) übertragen wurde, auf **S_{OA}** übertragen, insbesondere auf den Teil von **S_{OA}** übertragen, der einer Verdichtung, bevorzugt der Verdichtung in Schritt (c), zugeführt wird, wobei es sich um eine Vorverdichtung von **S_{OA}** oder die Verdichtung von **S_{OA}** zu **S_{OA1}** handeln kann. Bevorzugt handelt es sich dabei um die erste Verdichtung, der der Strom **S_{OA},** nachdem der die Kolonne **RD_{A}** verlassen hat, unterworfen wird. Dies ermöglicht es, einen Teil der noch von dem mindestens einen Teil **S_{OA2}** gespeicherte Restenergie bzw. Restwärme im Prozess einzusetzen, in diesem Fall zur Erwärmung von zu verdichtendem **S_{OA}.**

Andere, bevorzugte zusätzliche Senken für die Energie, bevorzugt Wärme, in dem mindestens einen Teil von **S_{OA2},** sind weiter unten beschrieben (siehe Absatz 4.3).

### 4.2 Verfahren zur Herstellung mindestens eines Alkalimetallalkoholats

Das im Verfahren zur Aufarbeitung eines Gemisches **G** gemäß der Erfindung eingesetzte Gemisch **G** ist ein einer Reaktionskolonne zur Herstellung von Alkalimetallalkoholaten entnommenes Wasser/Methanol-Gemisch.

Die vorliegende Erfindung betrifft somit ein Verfahren zur Herstellung mindestens eines Alkalimetallalkoholats der Formel M_{A}OR, wobei R Methyl ist, und wobei M_{A} ein Metall ausgewählt aus Natrium, Kalium, bevorzugt Natrium ist.

ROH ist demnach Methanol.

### 4.2.1 Schritt (α1)

Im Schritt (α1) des Verfahrens gemäß der Erfindung wird ein Eduktstrom **S_{AE1}** umfassend ROH mit einem Eduktstrom **S_{AE2}** umfassend M_{A}OH im Gegenstrom in einer Reaktivrektifikationskolonne **RR_{A}** zu einem Rohprodukt **RP_{A}** umfassend M_{A}OR, Wasser, ROH, M_{A}OH umgesetzt.

Als "Reaktivrektifikationskolonne" wird erfindungsgemäß eine Rektifikationskolonne definiert, in der zumindest in einigen Teilen die Umsetzung nach Schritt (α1) oder Schritt (α2) des erfindungsgemäßen Verfahrens ablaufen. Sie kann auch als "Reaktionskolonne" abgekürzt werden.

In Schritt (α1) wird am unteren Ende von **RR_{A}** ein Sumpfproduktstrom **S_{AP}** umfassend ROH und M_{A}OR entnommen. Am oberen Ende von **RR_{A}** wird ein Brüdenstrom **S_{AB}** umfassend Wasser und ROH entnommen.

M_{A} ist ausgewählt aus Natrium, Kalium, und bevorzugt Natrium.

Der Eduktstrom **S_{AE1}** umfasst ROH. In einer bevorzugten Ausführungsform liegt der Massenanteil von ROH in **S_{AE1}** bei ≥ 95 Gew.-%, noch bevorzugter bei ≥ 99 Gew.-%, wobei **S_{AE1}** ansonsten insbesondere Wasser aufweist.

Der in Schritt (α1) als Eduktstrom **S_{AE1}** eingesetzte Alkohol ROH kann auch handelsüblicher Alkohol mit einem Alkoholmassenanteil von mehr als 99.8 Gew.-% und einem Massenanteil an Wasser von bis zu 0.2 Gew.-% sein.

Der Eduktstrom **S_{AE1}** wird bevorzugt dampfförmig zugegeben.

Der Eduktstrom **S_{AE2}** umfasst M_{A}OH. In einer bevorzugten Ausführungsform umfasst **S_{AE2}** neben M_{A}OH mindestens eine weitere Verbindung ausgewählt aus Wasser, ROH. Noch bevorzugter umfasst **S_{AE2}** neben M_{A}OH Wasser, dann handelt es sich bei **S_{AE2}** um eine wässrige Lösung von M_{A}OH.

Wenn der Eduktstrom **S_{AE2}** M_{A}OH und Wasser umfasst, liegt der Massenanteil von M_{A}OH, bezogen auf das Gesamtgewicht der wässrigen Lösung, welche **S_{AE2}** bildet, insbesondere im Bereich von 10 bis 75 Gew.-%, bevorzugt von 15 bis 54 Gew.-%, bevorzugter von 30 bis 53 Gew.-% und besonders bevorzugt von 40 bis 52 Gew.-%.

Wenn der Eduktstrom **S_{AE2}** M_{A}OH und ROH umfasst, liegt der Massenanteil von M_{A}OH in ROH, bezogen auf das Gesamtgewicht der Lösung, welche **S_{AE2}** bildet, insbesondere im Bereich von 10 bis 75 Gew.-%, bevorzugt von 15 bis 54 Gew.-%, bevorzugter von 30 bis 53 Gew.-%, und besonders bevorzugt von 40 bis 52 Gew.-%.

In dem besonderen Fall, in dem der Eduktstrom **S_{AE2}** neben M_{A}OH sowohl Wasser als auch ROH umfasst, ist es besonders bevorzugt, dass der Massenanteil von M_{A}OH in ROH und Wasser, bezogen auf das Gesamtgewicht der Lösung, welche **S_{AE2}** bildet, insbesondere im Bereich von 10 bis 575 Gew.-%, bevorzugt von 15 bis 54 Gew.-%, bevorzugter von 30 bis 53 Gew.-%, und besonders bevorzugt von 40 bis 52 Gew.-% liegt.

Schritt (α1) wird in einer Reaktivrektifikationskolonne (oder "Reaktionskolonne") **RR_{A}** durchgeführt.

Schritt (α2), der unten weiter erklärt wird, wird in einer Reaktivrektifikationskolonne (oder "Reaktionskolonne") **RR_{B}** durchgeführt.

Bevorzugt enthält die Reaktionskolonne **RR_{A}** bzw. **RR_{B}** Einbauten. Geeignete Einbauten sind zum Beispiel Böden, strukturierte Packungen oder unstrukturierte Packungen. Wenn die Reaktionskolonne **RR_{A}** bzw. **RR_{B}** Böden enthält, so sind Glockenböden, Ventilböden, Tunnelböden, Thormann-Böden, Kreuzschlitzglockenböden oder Siebböden geeignet. Wenn die Reaktionskolonne **RR_{A}** bzw. **RR_{B}** Böden enthält, so werden vorzugsweise solche Böden gewählt, bei denen maximal 5 Gew.-%, bevorzugt weniger als 1 Gew.-% der Flüssigkeit durch die jeweiligen Böden durchregnen. Die zur Minimierung des Durchregnens der Flüssigkeit erforderlichen konstruktiven Maßnahmen sind dem Fachmann geläufig. Bei Ventilböden werden zum Beispiel besonders dicht schließende Ventilbauarten gewählt. Durch Verringerung der Zahl der Ventile lässt sich zudem die Dampfgeschwindigkeit in den Bodenöffnungen auf das Doppelte des Wertes, der üblicherweise eingestellt wird, erhöhen. Bei Einsatz von Siebböden ist es besonders günstig, die Durchmesser der Bodenöffnungen zu verringern und die Zahl der Öffnungen beizubehalten oder noch zu vergrößern.

Bei Einsatz von strukturierten oder unstrukturierten Packungen sind strukturierte Packungen im Hinblick auf die gleichmäßige Verteilung der Flüssigkeit bevorzugt.

Bei Kolonnen mit unstrukturierten Packungen, insbesondere mit Füllkörpern, und bei Kolonnen mit strukturierten Packungen kann die gewünschte Charakteristik der Flüssigkeitsverteilung dadurch erzielt werden, dass in dem dem Kolonnenmantel benachbarten Randbereich des Kolonnenquerschnitts, der etwa 2 bis 5 % des gesamten Kolonnenquerschnitts entspricht, die Flüssigkeitsberieselungsdichte gegenüber den übrigen Querschnittsbereichen um bis zu 100 %, bevorzugt um 5 bis 15 %, verringert wird. Dies lässt sich zum Beispiel durch die gezielte Verteilung der Abtropfstellen der Flüssigkeitsverteiler oder deren Bohrungen mit einfachen Mitteln erreichen.

Das erfindungsgemäße Verfahren kann sowohl kontinuierlich als auch diskontinuierlich durchgeführt werden. Bevorzugt wird es kontinuierlich durchgeführt.

"Umsetzung eines Eduktstroms **S_{AE1}** umfassend ROH mit einem Eduktstrom **S_{AE2}** umfassend M_{A}OH im Gegenstrom" wird erfindungsgemäß insbesondere dadurch gewährleistet, dass die Zulaufstelle mindestens eines Teils des Eduktstroms **S_{AE1}** umfassend ROH in Schritt (α1) an der Reaktionskolonne **RR_{A}** unterhalb der Zulaufstelle des Eduktstroms **S_{AE2}** umfassend M_{A}OH liegt. Die Reaktionskolonne **RR_{A}** umfasst vorzugsweise mindestens 2, insbesondere 15 bis 40 theoretische Stufen zwischen der Zulaufstelle des Eduktstroms **S_{AE1}** und der Zulaufstelle des Eduktstroms **S_{AE2}.**

Die Reaktionskolonne **RR_{A}** kann als reine Abtriebskolonne betrieben werden. Dann wird im unteren Bereich der Reaktionskolonne **RR_{A}** dampfförmig der Eduktstrom **S_{AE1}** umfassend ROH zugeführt. Schritt (α1) umfasst auch den Fall, dass ein Teil des Eduktstroms **S_{AE1}** umfassend ROH zwar unterhalb der Zulaufstelle des Eduktstroms **S_{AE2}** umfassend Alkalilauge M_{A}OH, aber dennoch am oberen Ende oder im Bereich des oberen Endes der Reaktionskolonne **RR_{A}** dampfförmig zugegeben wird. Dadurch können die Abmessungen im unteren Bereich der Reaktionskolonne RR_{A} verringert werden. Wenn ein Teil des Eduktstroms **S_{AE1}** umfassend ROH, nämlich

Methanol, am oberen Ende oder im Bereich des oberen Endes der Reaktionskolonne **RR_{A}** insbesondere dampfförmig zugegeben wird, so wird nur eine Teilmenge von 10 bis 70 Gew.-%, bevorzugt von 30 bis 50 Gew.-% (jeweils bezogen auf die Gesamtmenge des in Schritt (α1) eingesetzten Alkohols ROH) am unteren Ende der Reaktionskolonne **RR_{A}** eingespeist und die restliche Teilmenge in einem Einzelstrom oder auf mehrere Teilströme verteilt, bevorzugt 1 bis 10 theoretische Stufen, besonders bevorzugt 1 bis 3 theoretische Stufen unterhalb der Zulaufstelle des Eduktstroms **S_{AE2}** umfassend M_{A}OH dampfförmig zugegeben.

In der Reaktionskolonne **RR_{A}** setzt sich dann der Eduktstrom **S_{AE1}** umfassend ROH mit dem Eduktstrom **S_{AE2}** umfassend M_{A}OH gemäß der vorstehend beschriebenen Reaktion **<1>** zu M_{A}OR und H₂O um, wobei, da es sich um eine Gleichgewichtsreaktion handelt, diese Produkte in Mischung mit den Edukten ROH und M_{A}OH vorliegen. Demnach wird in Schritt (α1) ein Rohprodukt **RP_{A}** in der Reaktionskolonne **RR_{A}** erhalten, welches neben den Produkten M_{A}OR und Wasser auch noch ROH und M_{A}OH umfasst.

Am unteren Ende von **RR_{A}** erhält und entnimmt man dann den Sumpfproduktstrom **S_{AP}** umfassend ROH und M_{A}OR.

Am oberen Ende von **RR_{A},** bevorzugt am Kolonnenkopf von **RR_{A},** entnimmt man noch Wasser enthaltenden Alkoholstrom, vorstehend als "Brüdenstrom **S_{AB}** umfassend Wasser und ROH" bezeichnet.

Dieser Brüdenstrom **S_{AB}** umfassend Wasser und ROH wird im Schritt (β) mindestens teilweise als Gemisch **G** in Schritt (a) des Verfahrens gemäß der Erfindung eingesetzt. Ein Teil des bei der Destillation in Schritt (a) im Strom **S_{OA}** gewonnenen Alkohols kann der Reaktionskolonne **RR_{A}** als Eduktstrom **S_{AE1}** zugeführt werden.

In einer bevorzugten Ausführungsform des erfindungsgemäßen Verfahrens wird ein Teil von **S_{OA}** in Schritt (α1) als Eduktstrom **S_{AE1}** und, falls Schritt (α2) durchgeführt wird, alternativ oder zusätzlich in Schritt (α2) als Eduktstrom **S_{BE1}** eingesetzt.

In einer bevorzugteren Ausführungsform des erfindungsgemäßen Verfahrens werden 5 bis 95 Gew.-%, bevorzugt 10 bis 90 Gew.-%, bevorzugter 20 bis 80 Gew.-%, noch bevorzugter 30 bis 70 Gew.-% des Brüdenstromes **S_{OA}** als Eduktstrom **S_{AE1}** bzw. falls Schritt (α2) durchgeführt wird, alternativ oder zusätzlich in Schritt (α2) als Eduktstrom **S_{BE1}** eingesetzt.

In dieser bevorzugten Ausführungsform ist es vorteilhaft, den Teil des Stromes **S_{OA},** der als Eduktstrom **S_{AE1}** bzw. als Eduktstrom **S_{BE1}** eingesetzt wird, zu verdichten.

Die Menge des vom Eduktstrom **S_{AE1}** umfassten Alkohols ROH wird vorzugsweise so gewählt, dass dieser gleichzeitig als Lösungsmittel für das im Sumpfproduktstrom **S_{AP}** erhaltene Alkalialkoholat M_{A}OR dient. Vorzugsweise wird die Menge des Alkohols ROH im Eduktstrom **S_{AE1}** so gewählt, dass im Sumpf der Reaktionskolonne die gewünschte Konzentration der Alkalialkoholat-Lösung vorliegt, die als Sumpfproduktstrom **S_{AP}** umfassend ROH und M_{A}OR entnommen wird.

In einer bevorzugten Ausführungsform des erfindungsgemäßen Verfahrens, und insbesondere in den Fällen, in denen **S_{AE2}** neben M_{A}OH auch Wasser umfasst, beträgt das Verhältnis des Gesamtgewichts (Massen; Einheit: kg) an in Schritt (α1) als Eduktstrom **S_{AE1}** eingesetztem Alkohol ROH zum Gesamtgewicht (Massen; Einheit: kg) an in Schritt (α1) als Eduktstrom **S_{AE2}** eingesetzten M_{A}OH 4 : 1 bis 50 : 1, bevorzugter 8 : 1 bis 48 : 1, noch bevorzugter 10 : 1 bis 45 : 1, noch mehr bevorzugter 20 : 1 bis 40 : 1.

Die Reaktionskolonne **RR_{A}** wird mit oder ohne, vorzugsweise mit Rücklauf betrieben.

"Mit Rücklauf" bedeutet, dass der am oberen Ende der jeweiligen Kolonne, in Schritt (α1) der Reaktionskolonne **RR_{A},** in Schritt (α2) der Reaktionskolonne **RR_{B},** entnommene Brüdenstrom **S_{AB}** bzw. **S_{BB}** umfassend Wasser und ROH nicht vollständig abgeführt wird. Im Schritt (β) wird dann der betreffende Brüdenstrom **S_{AB}** bzw. **S_{BB}** also nicht vollständig als Gemisch **G** eingesetzt, sondern mindestens teilweise, bevorzugt teilweise, wieder als Rücklauf der jeweiligen Kolonne, in Schritt (α1) der Reaktionskolonne **RR_{A},** in Schritt (α2) der Reaktionskolonne **RR_{B},** zugeführt. In den Fällen, in denen ein solcher Rücklauf eingestellt wird, beträgt das Rücklaufverhältnis dabei bevorzugt 0.01 bis 1, bevorzugter 0.02 bis 0.9, noch bevorzugter 0.03 bis 0.34, besonderes bevorzugt 0.04 bis 0.27 und ganz besonders bevorzugt 0.05 bis 0.24.

Ein Rücklauf kann dadurch eingestellt werden, dass am Kopf der jeweiligen Kolonne ein Kondensator angebracht wird. In Schritt (α1) wird dazu insbesondere an der Reaktionskolonne **RR_{A}** ein Kondensator **K_{RRA}** angebracht. In Schritt (α2) wird dazu insbesondere an der Reaktionskolonne **RR_{B}** ein Kondensator **K_{RRB}** angebracht. In dem jeweiligen Kondensator wird der jeweilige Brüdenstrom **S_{AB}** bzw. **S_{BB}** mindestens teilweise kondensiert und der jeweiligen Kolonne, in Schritt (α1) der Reaktionskolonne **RR_{A}** bzw. **RR_{B}** wieder zugeführt wird.

In der Ausführungsform, in der an der Reaktionskolonne **RR_{A}** ein Rücklauf eingestellt wird, kann der in Schritt (α1) als Eduktstrom **S_{AE2}** eingesetzte M_{A}OH auch mindestens teilweise mit dem Rücklaufstrom vermischt werden und die resultierende Mischung so dem Schritt (α1) zugeführt werden.

Der Schritt (α1) wird insbesondere bei einer Temperatur im Bereich von 45 °C bis 150 °C, bevorzugt 47 °C bis 120 °C, bevorzugter 60 °C bis 110 °C, und bei einem Druck von 0.5 bar abs. bis 40 bar abs., bevorzugt im Bereich von 0.7 bar abs. bis 5 bar abs., bevorzugter im Bereich von 0.8 bar abs. bis 4 bar abs., bevorzugter im Bereich von 0.9 bar abs. bis 3.5 bar abs., noch bevorzugter bei 1.0 bar abs. bis 3 bar abs. durchgeführt.

Die Reaktionskolonne **RR_{A}** umfasst in einer bevorzugten Ausführungsform mindestens einen Verdampfer, der insbesondere aus Zwischenverdampfern **V_{ZA}** und Sumpfverdampfern **V_{SA}** ausgewählt ist. Die Reaktionskolonne **RR_{A}** umfasst besonders bevorzugt mindestens einen Sumpfverdampfer **V_{SA}.**

Als "Zwischenverdampfer" **V**_{Z} werden erfindungsgemäß Verdampfer bezeichnet, die sich oberhalb des Sumpfs der jeweiligen Kolonne, insbesondere oberhalb des Sumpfs der Reaktionskolonne **RR_{A}** bzw. **RR_{B}** (dann als "**V**_{ZA}" bzw. "**V**_{ZB}" bezeichnet) oder oberhalb des Sumpfes der Rektifikationskolonne **RD_{A}** (dann als "**V**_{ZRD}" bezeichnet) befinden. Im Falle von **RR_{A}** bzw. **RR_{B}** wird in ihnen insbesondere Rohprodukt **RP_{A}** bzw. **RP_{B}** verdampft, das der Kolonne als Seitenstrom **S_{ZAA}** bzw. **S_{ZBA}** entnommen wird.

Als "Sumpfverdampfer" **V_{S}** werden erfindungsgemäß Verdampfer bezeichnet, die den Sumpf der jeweiligen Kolonne, insbesondere den Sumpf der Reaktionskolonne **RR_{A}** bzw. **RR_{B}** bzw. der in der bevorzugten Ausführungsform verwendeten und weiter unten näher beschriebenen **RR_{C}** (dann als "**V**_{SA}" oder "**V**_{SA'}" bzw. "**V**_{SB}" oder "**V**_{SB'}" bzw. "**V**_{SC}" oder "**V**_{SC'}" bezeichnet) oder den Sumpf der Rektifikationskolonne **RD**_{A} (dann als "**V**_{SRD}" oder "**V**_{SRD'}" bezeichnet) beheizen. Im Falle von **RR_{A}** bzw. **RR_{B}** wird in ihnen insbesondere mindestens ein Teil des Sumpfproduktstroms **S**_{AP} bzw. **S**_{BP} verdampft. Im Falle von **RR_{C}** wird in ihnen insbesondere Sumpfproduktstrom **S**_{CP} verdampft. Im Falle von **RD_{A}** wird in ihnen insbesondere Sumpfproduktstrom **S**_{UA} oder ein Teil von **S**_{UA}, **S**_{UA1}, verdampft.

Ein Verdampfer ist üblicherweise außerhalb der jeweiligen Reaktionskolonne oder Rektifikationskolonne angeordnet. Da in Verdampfern Energie, insbesondere Wärme, von einem Strom auf einen anderen übertragen wird, sind sie Wärmeüberträger **WT.** Über einen Abzug aus der Kolonne wird das zu verdampfende Gemisch abgezogen und dem mindestens einen Verdampfer zugeführt. Im Falle der Reaktionskolonne **RR**_{A} bzw. **RR**_{B} wird bei der Zwischenverdampfung des Rohprodukts **RP**_{A} bzw. **RP**_{B}, dieses abgezogen und dem mindestens einen Zwischenverdampfer **V**_{ZA} bzw. **V**_{ZB} zugeführt.

Im Falle der Rektifikationskolonne **RD**_{A} wird bei der Zwischenverdampfung mindestens ein Seitenstrom **S**_{ZA} aus **RD**_{A} entnommen ("abgezogen") und dem mindestens einen Zwischenverdampfer **V**_{ZRD} zugeführt.

Im Falle der Rektifikationskolonne **RD**_{A} wird bei der Sumpfverdampfung mindestens ein Strom **S**_{UA} aus **RD**_{A} entnommen ("abgezogen") und mindestens ein Teil, bevorzugt ein Teil, dem mindestens einen Sumpfverdampfer **V**_{SRD} zugeführt.

Über mindestens einen Zulauf wird das verdampfte Gemisch gegebenenfalls mit einem Restanteil Flüssigkeit wieder in die jeweilige Kolonne zurückgeführt. Wenn der Verdampfer ein Zwischenverdampfer ist, es sich also insbesondere um einen Zwischenverdampfer **V_{ZA}** bzw. **V_{ZB}** bzw. **V_{ZRD}** handelt, ist der Abzug, über den das jeweilige Gemisch abgezogen und dem Verdampfer zugeführt wird, ein Seitenabzug und der Zulauf, über den das verdampfte Gemisch der jeweiligen Kolonne wieder zugeführt wird, ein Seitenzulauf. Wenn der Verdampfer ein Sumpfverdampfer ist, also den Kolonnensumpf beheizt, es sich also insbesondere um einen Sumpfverdampfer **V_{SA}** bzw. **V_{SB}** bzw. **V_{SRD}** handelt, so wird zumindest ein Teil des Sumpfabzugsstroms, insbesondere **S_{AP}** bzw. **S_{BP},** dem Sumpfverdampfer zugeführt, verdampft und im Bereich des Sumpfs wieder in die jeweilige Kolonne zurückgeführt. Alternativ ist es jedoch auch möglich, zum Beispiel auf einem geeigneten Boden bei Einsatz eines Zwischenverdampfers oder im Sumpf der jeweiligen Kolonne Rohre auszubilden, die von dem Wärmeträger, z.B. dem jeweiligen verdichteten Brüdenstrom **S_{OA11}** bzw. **S_{OA2}** (wenn **V_{S}** bzw. **V_{Z}** sich an der Rektifikationskolonne **RD_{A}** befinden) bzw. einem Wärmemedium **W₁** durchströmt werden. In diesem Fall erfolgt die Verdampfung auf dem Boden bzw. im Sumpf der Kolonne. Bevorzugt ist es jedoch, den Verdampfer außerhalb der jeweiligen Kolonne anzuordnen.

Geeignete Verdampfer, die als Zwischenverdampfer und Sumpfverdampfer eingesetzt werden können, sind zum Beispiel Naturumlaufverdampfer, Zwangsumlaufverdampfer, Zwangsumlaufverdampfer mit Entspannung, Kesselverdampfer, Fallfilmverdampfer oder Dünnschichtverdampfer. Als Wärmeübertrager für den Verdampfer wird bei Naturumlaufverdampfern und Zwangsumlaufverdampfern üblicherweise ein Rohrbündel oder Plattenapparat eingesetzt. Bei Einsatz eines Rohrbündelüberträgers kann der Wärmeträger, z.B. der verdichtete Brüdenstrom **S_{OA11}** bzw. **S_{OA2}** in **V_{SRD}** bzw. **V_{ZRD}** an der Rektifikationskolonne **RD_{A}** bzw. das Wärmemedium **W₁** entweder durch die Rohre strömen und das zu verdampfende Gemisch die Rohre umströmen oder aber der Wärmeträger, z.B. der verdichtete Brüdenstrom **S_{OA11}** bzw. **S_{OA2}** in **V_{SRD}** bzw. **V_{ZRD}** an der Rektifikationskolonne **RD_{A}** bzw. das Wärmemedium **W₁** umströmt die Rohre und das zu verdampfende Gemisch durchströmt die Rohre. Bei einem Fallfilmverdampfer wird das zu verdampfende Gemisch üblicherweise als dünner Film auf der Innenseite eines Rohres zugegeben und das Rohr wird von außen beheizt. Im Unterschied zu einem Fallfilmverdampfer ist in einem Dünnschichtverdampfer zusätzlich ein Rotor mit Wischern vorgesehen, der die zu verdampfende Flüssigkeit auf der Innenwand des Rohres zu einem dünnen Film verteilt.

Neben den genannten kann aber auch jede beliebige andere, dem Fachmann bekannte Verdampferbauart, die sich zum Einsatz an einer Rektifikationskolonne eignet, eingesetzt werden.

Wenn der Verdampfer, der z.B. mit dem verdichteten Brüdenstrom **S_{OA11}** bzw. das Wärmemedium **W₁** als Heizdampf betrieben wird, ein Zwischenverdampfer ist, ist es bevorzugt, wenn der Zwischenverdampfer im Abtriebsteil der Rektifikationskolonne **RD_{A}** im Bereich zwischen der Zulaufstelle des Gemischs **G** und oberhalb des Kolonnensumpfes bzw. im Falle der Reaktionskolonnen **RR_{A}** bzw. **RR_{B}** unterhalb der Zulaufstelle des Eduktstroms **S_{AE2}** bzw. **S_{BE2}** angeordnet ist. Dadurch kann ein überwiegender Teil der Heizenergie durch den Zwischenverdampfer eingebracht werden. So ist es zum Beispiel möglich, über 80% der Energie über den Zwischenverdampfer einzubringen. Erfindungsgemäß wird der Zwischenverdampfer vorzugsweise so angeordnet und/oder gestaltet, dass mit diesem mehr als 10%, insbesondere mehr als 20% der für die Destillation benötigten Gesamtenergie eingebracht werden.

Bei Einsatz eines Zwischenverdampfers ist es insbesondere vorteilhaft, wenn der Zwischenverdampfer so angeordnet ist, dass die jeweilige Rektifikationskolonne bzw. Reaktionskolonne unterhalb des Zwischenverdampfers 1 bis 50 theoretische Stufen aufweist und oberhalb des Zwischenverdampfers 1 bis 200 theoretische Stufen. Insbesondere ist es bevorzugt, wenn die Rektifikationskolonne bzw. Reaktionskolonne unterhalb des Zwischenverdampfers 2 bis 10 theoretische Stufen aufweist und oberhalb des Zwischenverdampfers 20 bis 80 theoretische Stufen.

Der Seitenabzugsstrom, über den das Gemisch aus der Rektifikationskolonne bzw. Reaktionskolonne dem Zwischenverdampfer **V_{Z}** zugeführt wird, und der Seitenzulauf, über den das verdampfte Gemisch aus dem Zwischenverdampfer **V_{Z}** wieder der jeweiligen Rektifikationskolonne bzw. Reaktionskolonne zugeführt wird, können zwischen den gleichen Böden der Kolonne positioniert sein. Es ist jedoch auch möglich, dass Seitenabzug und Seitenzulauf auf unterschiedlicher Höhe liegen.

In einem solchen Zwischenverdampfer **V_{ZA}** kann in der Reaktionskolonne **RR_{A}** befindliches, flüssiges Rohprodukt **RP_{A}** umfassend M_{A}OR, Wasser, ROH, M_{A}OH in den gasförmigen Zustand überführt werden, und so die Effizienz der Umsetzung gemäß Schritt (α1) des erfindungsgemäßen Verfahrens verbessert werden.

In einem solchen Zwischenverdampfer **V_{ZB}** kann in der Reaktionskolonne **RR_{B}** befindliches, flüssiges Rohprodukt **RP_{B}** umfassend M_{B}OR, Wasser, ROH, M_{B}OH in den gasförmigen Zustand überführt werden, und so die Effizienz der Umsetzung gemäß Schritt (α2) des erfindungsgemäßen Verfahrens verbessert werden.

Durch die Anordnung von einem oder mehreren Zwischenverdampfern **V_{ZA}** bzw. **V_{ZB}** im oberen Bereich der Reaktionskolonne **RR_{A}** können die Abmessungen im unteren Bereich der Reaktionskolonne **RR_{A}** verringert werden. Bei der Ausführungsform mit mindestens einem, bevorzugt mehreren Zwischenverdampfern **V_{ZA}** bzw. **V_{ZB}** ist es auch möglich, Teilströme des ROH in flüssiger Form im oberen Bereich der Reaktionskolonne **RR_{A}** zuzuführen.

In einer weiteren bevorzugten Ausführungsform wird Energie, bevorzugt Wärme, von mindestens einem Teil eines Stroms ausgewählt aus **S_{OA1}, S_{OA2},** insbesondere ausgewählt aus **S_{OA11}, S_{OA12}, S_{OA2},** bevorzugt ausgewählt aus **S_{OA11}, S_{OA2},** auf das Rohprodukt **RP_{A}** und, falls Schritt (α2) durchgeführt wird, alternativ oder zusätzlich auf das Rohprodukt **RP_{B}** übertragen.

"Übertragung von Energie, bevorzugt Wärme, von mindestens einem Teil von **S_{OA1}** auf das Rohprodukt **RP_{A}** und, falls Schritt (α2) durchgeführt wird, alternativ oder zusätzlich auf das Rohprodukt **RP_{B}"** umfasst auch die Übertragung von Energie, bevorzugt Wärme, von mindestens einem Strom ausgewählt aus **S_{OA11}, S_{OA12},** oder vom Strom **S_{OA1}** vor dessen Auftrennung in **S_{OA11}, S_{OA12},** auf das Rohprodukt **RP_{A}** und, falls Schritt (α2) durchgeführt wird, alternativ oder zusätzlich auf das Rohprodukt **RP_{B}.** Es umfasst auch die Übertragung von Energie von einem Teil von **S_{OA11}, S_{OA12}** auf das Rohprodukt **RP_{A}** und, falls Schritt (α2) durchgeführt wird, alternativ oder zusätzlich auf das Rohprodukt **RP_{B}.**

Dazu wird insbesondere ein Teil des betreffenden Stroms ausgewählt aus **S_{OA1}, S_{OA2}** oder ein Wärmeträger **W₁**, auf den vorher Energie von dem betreffenden Stroms ausgewählt aus **S_{OA1}, S_{OA2}** übertragen wurde, mindestens teilweise über einen Zwischenverdampfer **V_{ZA}** bzw. **V_{ZB}** geleitet und die Energie von dem betreffenden Stroms ausgewählt aus **S_{OA1}, S_{OA2}** bzw. **W₁** auf den per Seitenabzug auf **RR_{A}** bzw. **RR_{B}** abgezogenen Rohproduktstrom übertragen wird, insbesondere in dem der betreffende Stroms ausgewählt aus **S_{OA1}, S_{OA2}** bzw. **W₁** zur Beheizung des Verdampfers **V_{ZA}** bzw. **V_{ZB}** genutzt wird.

Sumpfverdampfer sind erfindungsgemäß am Sumpf der jeweiligen Rektifikationskolonne **RD_{A}** bzw. Reaktionskolonne **RR_{A}** bzw. **RR_{B}** bzw. **RR_{C}** angeordnet und werden dann als "**V_{SRD}**" oder "**V_{SRD'}**" bzw. "**V_{SA}**" oder "**V_{SA'}**" bzw. "**V_{SB}**" oder "**V_{SB'}**" bzw. "**V_{SC}**" oder "**V_{SC'}**" bezeichnet. In einem solchen Sumpfverdampfer kann ein in der jeweiligen Kolonne (insbesondere Reaktionskolonne **RR_{A}** bzw. **RR_{B}**) befindlicher Sumpfproduktstrom (insbesondere **S_{AP}** bzw. **S_{BP}**) geleitet und zum Beispiel ROH mindestens teilweise daraus entfernt werden. Im Falle von **S_{AP}** bzw. **S_{BP}** kann dadurch ein Sumpfproduktstrom **S_{AP*}** mit einem gegenüber **S_{AP}** erhöhten Massenanteil an M_{A}OR bzw. ein Sumpfproduktstrom **S_{BP*}** mit einem gegenüber **S_{BP}** erhöhten Massenanteil an M_{B}OR erhalten werden.

In Schritt (α1) des erfindungsgemäßen Verfahrens wird am unteren Ende der Reaktionskolonne **RR_{A}** ein Sumpfproduktstrom **S_{AP}** umfassend ROH und M_{A}OR entnommen.

Es ist bevorzugt, dass die Reaktionskolonne **RR_{A}** mindestens einen Sumpfverdampfer **V_{SA}** aufweist, über den Sumpfproduktstrom **S_{AP}** dann teilweise geleitet wird und ROH aus diesem teilweise entfernt wird, wodurch ein Sumpfproduktstrom **S_{AP*}** mit einem gegenüber **S_{AP}** erhöhten Massenanteil an M_{A}OR erhalten wird.

In einer anderen bevorzugten Ausführungsform wird deshalb zur Übertragung von Energie, bevorzugt Wärme, von mindestens einem Teil eines Stroms ausgewählt aus **S_{OA1}, S_{OA2},** insbesondere ausgewählt aus **S_{OA11}, S_{OA2},** auf das Rohprodukt **RP_{A}** und, falls Schritt (α2) durchgeführt, alternativ oder zusätzlich auf das Rohprodukt **RP_{B}**, wie folgt vorgegangen:
Es wird dann insbesondere ein Teil des betreffenden Stroms ausgewählt aus **S_{OA1}, S_{OA2}** oder ein Wärmeträger **W₁**, auf den vorher Energie von dem betreffenden Stroms ausgewählt aus **S_{OA1}, S_{OA2}** übertragen wurde, mindestens teilweise über einen Sumpfverdampfer **V_{SA}** bzw. **V_{SB}** geleitet und die Energie von dem betreffenden Stroms ausgewählt aus **S_{OA1}, S_{OA2}** bzw. **W₁** auf den Sumpfproduktstrom **S_{AP}** bzw. **S_{BP}** übertragen, insbesondere in dem der betreffende Stroms ausgewählt aus **S_{OA1}, S_{OA2}** bzw. **W₁** zur Beheizung des Verdampfers **V_{SA}** bzw. **V_{SB}** genutzt wird.

Der Massenanteil an M_{A}OR des Sumpfproduktstroms **S_{AP*}** ist dabei insbesondere gegenüber dem Massenanteil an M_{A}OR des Sumpfproduktstroms **S_{AP}** um mindestens 0.5 % erhöht, bevorzugt um ≥ 1 %, bevorzugter um ≥ 2 %, noch bevorzugter um ≥ 5 %,

Bevorzugt weist **S_{AP}** oder, falls mindestens einen Sumpfverdampfer **V_{SA}** eingesetzt wird, über den der Sumpfproduktstrom **S_{AP}** mindestens teilweise geleitet wird und ROH aus diesem mindestens teilweise entfernt wird, **S_{AP*}**, einen Massenanteil von M_{A}OR in ROH im Bereich von 1 bis 50 Gew.-%, bevorzugt 5 bis 35 Gew.-%, bevorzugter 15 bis 35 Gew.-%, am bevorzugtesten 20 bis 35 Gew.-% auf, jeweils bezogen auf die Gesamtmasse von **S_{AP}**.

Der Massenanteil an Restwasser in **S_{AP}** bzw. **S_{AP*}** liegt dabei bevorzugt bei < 1 Gew.-%, bevorzugt < 0.8 Gew.-%, bevorzugter < 0.5 Gew.-%, bezogen auf die Gesamtmasse von **S_{AP}**.

Der Massenanteil an Edukt M_{A}OH in **S_{AP}** bzw. **S_{AP*}** liegt dabei bevorzugt bei < 1 Gew.-%, bevorzugt < 0.8 Gew.-%, bevorzugter < 0.5 Gew.-%, bezogen auf die Gesamtmasse von **S_{AP}**.

### 4.2.2 Schritt (α2) (optional)

Schritt (α2) ist eine optionale Ausführungsform des Verfahrens gemäß der Erfindung. Dies bedeutet, dass im Rahmen der bevorzugten Ausführungsform des Verfahrens gemäß der Erfindung der Schritt (α2) durchgeführt wird oder nicht. Im optionalen Schritt (α2) wird, gleichzeitig mit und räumlich getrennt von Schritt (α1), ein Eduktstrom **S_{BE1}** umfassend ROH mit einem Eduktstrom **S_{BE2}** umfassend M_{B}OH im Gegenstrom in einer Reaktivrektifikationskolonne **RR_{B}** zu einem Rohprodukt **RP_{B}** umfassend M_{B}OR, Wasser, ROH, M_{B}OH umgesetzt.

In Schritt (α2) des erfindungsgemäßen Verfahrens wird am unteren Ende von **RR_{B}** ein Sumpfproduktstrom **S_{BP}** umfassend ROH und M_{B}OR entnommen. Am oberen Ende von **RR_{B}** wird ein Brüdenstrom **S_{BB}** umfassend Wasser und ROH entnommen.

M_{B} ist ausgewählt aus Natrium, Kalium, und bevorzugt Kalium.

Der Eduktstrom **S_{BE1}** umfasst ROH. In einer bevorzugten Ausführungsform liegt der Massenanteil von ROH in **S_{BE1}** bei ≥ 95 Gew.-%, noch bevorzugter bei ≥ 99 Gew.-%, wobei **S_{BE1}** ansonsten insbesondere Wasser aufweist.

Der in Schritt (α2) des erfindungsgemäßen Verfahrens als Eduktstrom **S_{BE1}** eingesetzte Alkohol ROH kann auch handelsüblicher Alkohol mit einem Alkoholmassenanteil von mehr als 99.8 Gew.-% und einem Massenanteil an Wasser von bis zu 0.2 Gew.-% sein.

Der Eduktstrom **S_{BE1}** wird bevorzugt dampfförmig zugegeben.

Der Eduktstrom **S_{BE2}** umfasst M_{B}OH. In einer bevorzugten Ausführungsform umfasst **S_{BE2}** neben M_{B}OH mindestens eine weitere Verbindung ausgewählt aus Wasser, ROH. Noch bevorzugter umfasst **S_{BE2}** neben M_{B}OH Wasser, dann handelt es sich bei **S_{BE2}** um eine wässrige Lösung von M_{B}OH.

Wenn der Eduktstrom **S_{BE2}** M_{B}OH und Wasser umfasst, liegt der Massenanteil von M_{B}OH, bezogen auf das Gesamtgewicht der wässrigen Lösung, welche **S_{BE2}** bildet, insbesondere im Bereich von 10 bis 75 Gew.-%, bevorzugt von 15 bis 54 Gew.-%, bevorzugter von 30 bis 53 Gew.-% und besonders bevorzugt von 40 bis 52 Gew.-%.

Wenn der Eduktstrom **S_{BE2}** M_{B}OH und ROH umfasst, liegt der Massenanteil von M_{B}OH in ROH, bezogen auf das Gesamtgewicht der Lösung, welche **S_{BE2}** bildet, insbesondere im Bereich von 10 bis 75 Gew.-%, bevorzugt von 15 bis 54 Gew.-%, bevorzugter von 30 bis 53 Gew.-%, und besonders bevorzugt von 40 bis 52 Gew.-%.

In dem besonderen Fall, in dem der Eduktstrom **S_{BE2}** neben M_{B}OH sowohl Wasser als auch ROH umfasst, ist es besonders bevorzugt, dass der Massenanteil von M_{B}OH in ROH und Wasser, bezogen auf das Gesamtgewicht der Lösung, welche **S_{BE2}** bildet, insbesondere im Bereich von 10 bis 75 Gew.-%, bevorzugt von 15 bis 54 Gew.-%, bevorzugter von 30 bis 53 Gew.-%, und besonders bevorzugt von 40 bis 52 Gew.-% liegt.

Schritt (α2) des erfindungsgemäßen Verfahrens wird in einer Reaktivrektifikationskolonne (oder "Reaktionskolonne") **RR_{B}** durchgeführt. Bevorzugte Ausführungsformen der Reaktionskolonne **RR_{B}** sind im Abschnitt 4.2.1 beschrieben.

"Umsetzung eines Eduktstroms **S_{BE1}** umfassend ROH mit einem Eduktstrom **S_{BE2}** umfassend M_{B}OH im Gegenstrom " wird erfindungsgemäß insbesondere dadurch gewährleistet, dass die Zulaufstelle mindestens eines Teils des Eduktstroms **S_{BE1}** umfassend ROH in Schritt (α2) an der Reaktionskolonne **RR_{B}** unterhalb der Zulaufstelle des Eduktstroms **S_{BE2}** umfassend M_{B}OH liegt. Die Reaktionskolonne **RR_{B}** umfasst vorzugsweise mindestens 2, insbesondere 15 bis 40 theoretische Stufen zwischen der Zulaufstelle des Eduktstroms **S_{BE1}** und der Zulaufstelle des Eduktstroms **S_{BE2}**.

Die Reaktionskolonne **RR_{B}** kann als reine Abtriebskolonne betrieben werden. Dann wird im unteren Bereich der Reaktionskolonne **RR_{B}** dampfförmig der Eduktstrom **S_{BE1}** umfassend ROH zugeführt. Schritt (α2) des erfindungsgemäßen Verfahrens umfasst auch den Fall, dass ein Teil des Eduktstroms **S_{BE1}** umfassend ROH zwar unterhalb der Zulaufstelle des Eduktstroms **S_{BE2}** umfassend Alkalilauge M_{B}OH, aber dennoch am oberen Ende oder im Bereich des oberen Endes der Reaktionskolonne **RR_{B}** dampfförmig zugegeben wird. Dadurch können die Abmessungen im unteren Bereich der Reaktionskolonne **RR_{B}** verringert werden. Wenn ein Teil des Eduktstroms **S_{BE1}** umfassend ROH, nämlich Methanol, am oberen Ende oder im Bereich des oberen Endes der Reaktionskolonne **RR_{B}** insbesondere dampfförmig zugegeben wird, so wird nur eine Teilmenge von 10 bis 70 Gew.-%, bevorzugt von 30 bis 50 Gew.-% (jeweils bezogen auf die Gesamtmenge des in Schritt (α2) eingesetzten Alkohols ROH) am unteren Ende der Reaktionskolonne **RR_{B}** eingespeist und die restliche Teilmenge in einem Einzelstrom oder auf mehrere Teilströme verteilt, bevorzugt 1 bis 10 theoretische Stufen, besonders bevorzugt 1 bis 3 theoretische Stufen unterhalb der Zulaufstelle des Eduktstroms **S_{BE2}** umfassend M_{B}OH dampfförmig zugegeben.

In der Reaktionskolonne **RR_{B}** setzt sich dann der Eduktstrom **S_{BE1}** umfassend ROH mit dem Eduktstrom **S_{BE2}** umfassend M_{B}OH gemäß der vorstehend beschriebenen Reaktion <1> zu M_{B}OR und H₂O um, wobei, da es sich um eine Gleichgewichtsreaktion handelt, diese Produkte in Mischung mit den Edukten ROH und M_{B}OH vorliegen. Demnach wird in Schritt (α2) des erfindungsgemäßen Verfahrens in der Reaktionskolonne **RR_{B}** ein Rohprodukt **RP_{B}** erhalten, welches neben den Produkten M_{B}OR und Wasser auch noch ROH und M_{B}OH umfasst.

Am unteren Ende von **RR_{B}** erhält und entnimmt man dann den Sumpfproduktstrom **S_{BP}** umfassend ROH und M_{B}OR.

Am oberen Ende von **RR_{B},** bevorzugt am Kolonnenkopf von **RR_{B},** entnimmt man noch Wasser enthaltenden Alkoholstrom, vorstehend als "Brüdenstrom **S_{BB}** umfassend Wasser und ROH" bezeichnet.

In Schritt (β) des Verfahrens gemäß der Erfindung wird mindestens ein Teil dieses Brüdenstroms **S_{BB}** umfassend Wasser und ROH insbesondere als Gemisch **G** im Schritt (a) des erfindungsgemäßen Verfahrens eingesetzt. Dabei wird er, mit **S_{AB}** vermischt oder nicht, also getrennt von **S_{AB}** als Gemisch **G** der Rektifikationskolonne **RD_{A}** zugeführt. Bevorzugt werden die Brüdenströme **S_{BB}** und **S_{AB}** vermischt und dann die Mischung als Gemisch **G** im Schritt (a) des erfindungsgemäßen Verfahrens eingesetzt.

Die Menge des vom Eduktstrom **S_{BE1}** umfassten Alkohols ROH wird vorzugsweise so gewählt, dass dieser gleichzeitig als Lösungsmittel für das im Sumpfproduktstrom **S_{BP}** erhaltene Alkalialkoholat M_{B}OR dient. Vorzugsweise wird die Menge des Alkohols ROH im Eduktstrom **S_{BE1}** so gewählt, dass im Sumpf der Reaktionskolonne die gewünschte Konzentration der Alkalialkoholat-Lösung vorliegt, die als Sumpfproduktstrom **S_{BP}** umfassend ROH und M_{B}OR entnommen wird.

In einer bevorzugten Ausführungsform des erfindungsgemäßen Verfahrens, und insbesondere in den Fällen, in denen **S_{BE2}** neben M_{B}OH auch Wasser umfasst, beträgt das Verhältnis des Gesamtgewichts (Massen; Einheit: kg) an in Schritt (α2) als Eduktstrom **S_{BE1}** eingesetztem Alkohol ROH zum Gesamtgewicht (Massen; Einheit: kg) an in Schritt (α2) als Eduktstrom **S_{BE2}** eingesetzten M_{B}OH 4 : 1 bis 50 : 1, bevorzugter 8 : 1 bis 48 : 1, noch bevorzugter 10 : 1 bis 45 : 1, noch mehr bevorzugter 20 : 1 bis 40 : 1.

Die Reaktionskolonne **RR_{B}** wird mit oder ohne, vorzugsweise mit Rücklauf betrieben.

"Mit Rücklauf" bedeutet, dass der am oberen Ende der jeweiligen Kolonne, in Schritt (α2) der Reaktionskolonne **RR_{B},** entnommene Brüdenstrom **S_{BB}** umfassend Wasser und ROH nicht vollständig abgeführt wird, im Schritt (β) des erfindungsgemäßen Verfahrens also nicht vollständig als Gemisch **G** in Schritt (a) eingesetzt wird, also der Rektifikationskolonne **RD_{A}** zugeführt wird, sondern mindestens teilweise, bevorzugt teilweise, wieder als Rücklauf der jeweiligen Kolonne, in Schritt (α2) der Reaktionskolonne **RR_{B},** zugeführt wird. In den Fällen, in denen ein solcher Rücklauf eingestellt wird, beträgt das Rücklaufverhältnis dabei bevorzugt 0.01 bis 0.99, bevorzugter 0.02 bis 0.9, noch bevorzugter 0.03 bis 0.34, besonderes bevorzugt 0.04 bis 0.27 und ganz besonders bevorzugt 0.05 bis 0.24.

In der Ausführungsform, in der an der Reaktionskolonne **RR_{B}** ein Rücklauf eingestellt wird, kann der in Schritt (α2) als Eduktstrom **S_{BE2}** eingesetzte M_{B}OH auch mindestens teilweise mit dem Rücklaufstrom vermischt werden und die resultierende Mischung so dem Schritt (α2) zugeführt werden.

Der optionale Schritt (α2) wird insbesondere bei einer Temperatur im Bereich von 45 °C bis 150 °C, bevorzugt 47 °C bis 120 °C, bevorzugter 60 °C bis 110 °C, und bei einem Druck von 0.5 bar abs. bis 40 bar abs., bevorzugt im Bereich von 0.7 bar abs. bis 5 bar abs., bevorzugter im Bereich von 0.8 bar abs. bis 4 bar abs., bevorzugter im Bereich von 0.9 bar abs. bis 3.5 bar abs., noch bevorzugter bei 1.0 bar abs. bis 3 bar abs. durchgeführt.

Die Reaktionskolonne **RR_{B}** umfasst in einer bevorzugten Ausführungsform mindestens einen Verdampfer, der insbesondere aus Zwischenverdampfern **V_{ZB}** und Sumpfverdampfern **V_{SB}** ausgewählt ist. Die Reaktionskolonne **RR_{B}** umfasst besonders bevorzugt mindestens einen Sumpfverdampfer **V_{SB}**.

In einem solchen Zwischenverdampfer **V_{ZB}** kann in der Reaktionskolonne **RR_{B}** befindliches, flüssiges Rohprodukt **RP_{B}** umfassend M_{B}OR, Wasser, ROH, M_{B}OH in den gasförmigen Zustand überführt werden, und so die Effizienz der Umsetzung gemäß Schritt (α2) des erfindungsgemäßen Verfahrens verbessert werden.

Durch die Anordnung von einem oder mehreren Zwischenverdampfern **V_{ZB}** im oberen Bereich der Reaktionskolonne **RR_{B}** können die Abmessungen im unteren Bereich der Reaktionskolonne **RR_{B}** verringert werden. Bei der Ausführungsform mit mindestens einem, bevorzugt mehreren Zwischenverdampfern **V_{ZB}** ist es auch möglich, Teilströme des ROH in flüssiger Form im oberen Bereich der Reaktionskolonne **RR_{B}** zuzuführen.

In Schritt (α2) des erfindungsgemäßen Verfahrens wird am unteren Ende der Reaktionskolonne **RR_{B}** ein Sumpfproduktstrom **S_{BP}** umfassend ROH und M_{B}OR entnommen.

Es ist bevorzugt, dass die Reaktionskolonne **RR_{B}** mindestens einen Sumpfverdampfer **V_{SB}** aufweist, über den Sumpfproduktstrom **S_{BP}** dann mindestens teilweise geleitet wird und ROH aus diesem mindestens teilweise entfernt wird, wodurch ein Sumpfproduktstrom **S_{BP*}** mit einem gegenüber **S_{BP}** erhöhten Massenanteil an M_{B}OR erhalten wird.

Der Massenanteil an M_{B}OR des Sumpfproduktstroms **S_{BP*}** ist dabei insbesondere gegenüber dem Massenanteil an M_{B}OR des Sumpfproduktstroms **S_{BP}** um mindestens 0.5 % erhöht, bevorzugt um ≥ 1 %, bevorzugter um ≥ 2 %, noch bevorzugter um ≥ 5 %.

Bevorzugt weist **S_{BP}** oder, falls mindestens einen Sumpfverdampfer **V_{SB}** eingesetzt wird, über den der Sumpfproduktstrom **S_{BP}** mindestens teilweise geleitet wird und ROH aus diesem mindestens teilweise entfernt wird, **S_{BP*}**, einen Massenanteil von M_{B}OR in ROH im Bereich 1 bis 50 Gew.-%, bevorzugt 5 bis 35 Gew.-%, bevorzugter 15 bis 35 Gew.-%, am bevorzugtesten 20 bis 35 Gew.-% auf, jeweils bezogen auf die Gesamtmasse von **S_{BP}**.

Der Massenanteil an Restwasser in **S_{BP}** bzw. **S_{BP*}** liegt dabei bevorzugt bei < 1 Gew.-%, bevorzugt < 0.8 Gew.-%, bevorzugter < 0.5 Gew.-%, bezogen auf die Gesamtmasse von **S_{BP}**.

Der Massenanteil an Edukt M_{B}OH in **S_{BP}** bzw. **S_{BP*}** liegt dabei bevorzugt bei < 1 Gew.-%, bevorzugt < 0.8 Gew.-%, bevorzugter < 0.5 Gew.-%, bezogen auf die Gesamtmasse von **S_{BP}**.

In den Ausführungsformen des vorliegenden Verfahrens, in denen auch Schritt (α2) ausgeführt wird, wird bevorzugt der Sumpfproduktstrom **S_{AP}** mindestens teilweise über einen Sumpfverdampfer **V_{SA}** geleitet und ROH aus **S_{AP}** mindestens teilweise entfernt, wodurch ein Sumpfproduktstrom **S_{AP*}** mit einem gegenüber **S_{AP}** erhöhten Massenanteil an M_{A}OR erhalten wird und/oder, bevorzugt und, der Sumpfproduktstrom **S_{BP}** mindestens teilweise über einen Sumpfverdampfer **V_{SB}** geleitet und ROH aus **S_{BP}** mindestens teilweise entfernt, wodurch ein Sumpfproduktstrom **S_{BP*}** mit einem gegenüber **S_{BP}** erhöhten Massenanteil an M_{B}OR erhalten wird.

Der Schritt (α2) des erfindungsgemäßen Verfahrens wird in den Ausführungsformen der vorliegenden Erfindung, in denen er durchgeführt wird, gleichzeitig mit und räumlich getrennt von Schritt (α1) durchgeführt. Die räumliche Trennung wird durch die Durchführung der Schritte (α1) und (α2) in den beiden Reaktionskolonnen **RR_{A}** und **RR_{B}** gewährleistet.

In einer vorteilhaften Ausgestaltung der Erfindung sind die Reaktionskolonnen **RR_{A}** und **RR_{B}** in einem Kolonnenmantel untergebracht, wobei die Kolonne mindestens teilweise durch mindestens eine Trennwand unterteilt ist. Eine solche mindestens eine Trennwand aufweisende Kolonne wird als "**TRD**" bezeichnet. Solche Trennwandkolonnen sind dem Fachmann bekannt und beispielsweise beschrieben in US 2,295,256, EP 0 122 367 A2, EP 0 126 288 A2, WO 2010/097318 A1 sowie von I. Dejanović, Lj. Matijašević, Ž. Olujić, *Chemical Engineering and Processing* **2010,** 49, 559-580. CN 105218315 A beschreibt ebenfalls Trennwandkolonnen, die bei der Rektifikation von Methanol eingesetzt werden.

In den für das erfindungsgemäße Verfahren in Frage kommenden Trennwandkolonnen sind die Trennwände bevorzugt bis zum Boden durchgezogen und durchspannen insbesondere bevorzugt mindestens ein Viertel, bevorzugter mindestens ein Drittel, noch bevorzugter mindestens die Hälfte, noch bevorzugter mindesten zwei Drittel, noch mehr bevorzugter mindestens drei Viertel der Kolonne der Länge nach. Sie teilen die Kolonne in mindestens zwei Reaktionsräume, in denen räumlich getrennte Reaktionen stattfinden können. Die durch die mindestens eine Trennwand geschaffenen Reaktionsräume können gleich oder unterschiedlich groß sein.

In dem jeweils von der Trennwand getrennten Bereichen können in dieser Ausführungsform die Sumpfproduktströme **S_{AP}** und **S_{BP}** getrennt entnommen werden und bevorzugt über den für jeden durch die mindestens eine Reaktionswand gebildeten Reaktionsraum angebrachten Sumpfverdampfer **V_{SA}** bzw. **V_{SB}** geleitet werden, in dem ROH aus **S_{AP}** bzw. **S_{BP}** mindestens teilweise entfernt wird, wodurch **S_{AP*}** bzw. **S_{BP*}** erhalten werden.

In einer bevorzugten Ausführungsform des erfindungsgemäßen Verfahrens sind demnach mindestens zwei, noch bevorzugter genau zwei, der Kolonnen ausgewählt aus Rektifikationskolonne **RD_{A},** Reaktionskolonne **RR_{A}** und, falls Schritt (α2) durchgeführt wird, die Reaktionskolonne **RR_{B}** in einem Kolonnenmantel untergebracht, wobei die Kolonnen mindestens teilweise durch eine bis zum Boden der Kolonne durchgezogene Trennwand voneinander getrennt sind.

Im Verbund aus Reaktionskolonne **RR_{A}** (bzw. in den Ausführungsformen, in denen Schritt (α2) durchgeführt wird, Reaktionskolonne **RR_{A}** und Reaktionskolonne **RR_{B}**) mit Rektifikationskolonne **RD_{A}** im erfindungsgemäßen Verfahren wird die Rektifikationskolonne **RD_{A}** vorzugsweise bei einem Druck betrieben, der so gewählt wird, dass das Druckgefälle zwischen den Kolonnen gering ist.

Im erfindungsgemäßen Verfahren wird der Alkohol ROH verbraucht, und insbesondere bei kontinuierlicher Verfahrensführung muss dieser deshalb mit frischem Alkohol ROH ersetzt werden. Die Zuführung des frischen Alkohols ROH erfolgt dabei insbesondere direkt als Eduktstrom **S_{AE1}** umfassend ROH in die Reaktionskolonne **RR_{A}** bzw. in den Ausführungsformen, in denen Schritt (α2) durchgeführt wird, in die Reaktionskolonnen **RR_{A}** und **RR_{B}**.

Im erfindungsgemäßen Verfahren ist es weiterhin bevorzugt, den ROH umfassende Brüdenstrom **S_{OA}** teilweise als Eduktstrom **S_{AE1}** in Schritt (α1) und gegebenenfalls als Eduktstrom **S_{BE1}** in Schritt (α2) einzusetzen. Alternativ oder zusätzlich kann der verdichtete Brüdenstrom **S_{OA1}** teilweise als Eduktstrom **S_{AE1}** in Schritt (α1) und gegebenenfalls als Eduktstrom **S_{BE1}** in Schritt (α2) eingesetzt werden. In dieser bevorzugten Ausführungsform ist es noch bevorzugter, wenn der frische Alkohol ROH der Rektifikationskolonne **RD_{A}** zugegeben wird.

Wenn der frische Alkohol ROH der Rektifikationskolonne **RD_{A}** zugegeben wird, wird er bevorzugt entweder im Verstärkungsteil der Rektifikationskolonne **RD_{A}** oder direkt am Kopf der Rektifikationskolonne **RD_{A}** zugeführt. Die optimale Zulaufstelle ist abhängig vom Wassergehalt des eingesetzten frischen Alkohols und andererseits von dem gewünschten Restwassergehalt im Brüdenstrom **S_{OA}**. Je höher der Wasseranteil im eingesetzten Alkohol und je höher die Reinheitsanforderung im Brüdenstrom **S_{OA}** ist, um so günstiger ist ein Zulauf einige theoretische Stufen unterhalb des Kopfes der Rektifikationskolonne **RD_{A}**. Bevorzugt sind bis zu 20 theoretische Stufen unterhalb des Kopfes der Rektifikationskolonne **RD_{A}** und insbesondere 1 bis 5 theoretische Stufen.

Wenn der frische Alkohol ROH der Rektifikationskolonne **RD_{A}** zugegeben wird, wird er bei Temperaturen bis hin zum Siedepunkt, bevorzugt bei Raumtemperatur, am Kopf der Rektifikationskolonne **RD_{A}** zugegeben. Hierbei kann für den frischen Alkohol ein eigener Zulauf vorgesehen sein oder aber bei Rückführung eines Teils des am Kopf der Rektifikationskolonne **RD_{A}** entnommenen Alkohols nach der Kondensation mit diesem gemischt und gemeinsam in die Rektifikationskolonne **RD_{A}** zugeführt werden. In diesem Fall ist es besonders bevorzugt, wenn der frische Alkohol in einen Kondensatbehälter, in dem der aus dem Brüdenstrom **S_{OA}** kondensierte Alkohol gesammelt wird, zugegeben wird.

Wie oben beschrieben sind in einer vorteilhaften Ausgestaltung der Erfindung mindestens zwei der Kolonnen ausgewählt aus Rektifikationskolonne **RD_{A},** Reaktionskolonne **RR_{A}** und, falls Schritt (α2) durchgeführt wird, die Reaktionskolonne **RR_{B}** in einem Kolonnenmantel untergebracht, wobei die Kolonnen mindestens teilweise jeweils durch eine bis zum Boden der Kolonne durchgezogene Trennwand voneinander getrennt sind. In der vorbeschriebenen bevorzugten Ausführungsform, in der Schritt (α2) durchgeführt wird, werden diese demnach durch zwei Trennwände voneinander getrennt, wobei die zwei Trennwände bis zum Boden der Kolonne durchgezogen sind.

In dieser bevorzugten Ausführungsform wird insbesondere in einem Teil der **TRD** die Reaktion zum Rohprodukt **RP_{A}** gemäß Schritt (α1) bzw. den Rohprodukten **RP_{A}** und **RP_{B}** gemäß Schritten (α1) und (α2) durchgeführt, wobei der Eduktstrom **S_{AE2}** und gegebenenfalls der Eduktstrom **S_{BE2}** zwar unterhalb, aber in etwa auf der Höhe des oberen Endes der Trennwand zugegeben wird und der Eduktstrom **S_{AE1}** und gegebenenfalls der Eduktstrom **S_{BE1}** dampfförmig am unteren Ende aufgegeben wird. Das oberhalb der Zulaufstelle des Eduktstroms entstehende Alkohol/WasserGemisch verteilt sich dann oberhalb der Trennwand über den gesamten Kolonnenbereich, der als Verstärkungsteil der Rektifikationskolonne **RD_{A}** dient. Der zweite bzw. dritte, durch die Trennwand abgetrennte untere Teil der Kolonne ist der Abtriebsteil der Rektifikationskolonne **RD_{A}**. Die für die Destillation notwendige Energie wird dann über einen Verdampfer am unteren Ende des zweiten durch die Trennwand abgetrennten Teils der Kolonne zugeführt, wobei dieser Verdampfer konventionell beheizt werden kann oder mit einem Teil des verdichteten Brüdenstroms **S_{OA2}** beheizt werden kann. Wenn der Verdampfer konventionell beheizt wird, so kann zusätzlich ein Zwischenverdampfer vorgesehen werden, der mit einem Teil des verdichteten Brüdenstroms **S_{OA11}** beheizt wird.

In den Ausführungsformen, in denen ein Teil von **S_{OA}** als Eduktstrom **S_{AE1}** und/oder Eduktstrom **S_{BE1}** eingesetzt wird, wird **S_{OA}** insbesondere mit einem ersten Verdichter **VD_{AB2}** verdichtet ("vorverdichtet") wird, womit der Unterschied der Drücke innerhalb der Reaktionskolonnen **RR_{A}** und **RR_{B}** gegenüber dem Druck in **RD_{A}** berücksichtigt werden kann.

Alternativ oder zusätzlich kann in dieser bevorzugten Ausführungsform auch, anstatt des Verdichters **VD_{AB2}**, der der Rektifikationskolonne **RD_{A}** nachgeschaltet ist und in dem **S_{OA}** vorverdichtet wird, ein der Rektifikationskolonne **RD_{A}** vorgeschalteter Verdichter **VD_{AB1}** eingesetzt werden, über den das Gemisch **G,** bevor es in **RD_{A}** geleitet wird, geleitet wird.

Der übrige, also insbesondere nicht als Eduktstrom **S_{AE1}** und/oder Eduktstrom **S_{BE1}** eingesetzte Teil des mindestens einen Brüdenstromes **S_{OA}** wird, soweit er nicht als Rücklauf auf **RD_{A}** rückgeführt wird, in dieser bevorzugten Ausführungsform dann weiter zu **S_{OA1}** verdichtet. Erfindungsgemäß wird aus **S_{OA}** erst in jener Verdichterstufe **S_{OA1},** nach welcher die Aufteilung von **S_{OA1}** in **S_{OA11}** und **S_{OA12}** erfolgt, die im erfindungsgemäßen Schritt (d) eingesetzt werden. Diese Verdichtung, welche **S_{OA}** in **S_{OA1}** verdichtet, wird in den Abbildungen und Beispielen mit dem Verdichter **VD₁** (in den Abbildungen mit <401> bezeichnet) durchgeführt.

### 4.2.3 Schritt (β)

Im Schritt (β) des Verfahrens gemäß der Erfindung wird mindestens ein Teil des Brüdenstroms **S_{AB}**, und, wenn Schritt (α2) durchgeführt wird, mindestens ein Teil des Brüdenstroms **S_{BB}**, vermischt mit **S_{AB}** oder getrennt von **S_{AB}**, als Gemisch **G** in Schritt (a) des Verfahrens gemäß der Erfindung eingesetzt. Wenn Schritt (α2) durchgeführt wird, ist es bevorzugt, wenn der mindestens eine Teil des Brüdenstroms **S_{AB}**, und der mindestens eine Teil des Brüdenstroms **S_{BB}** gemischt werden und dann als Gemisch **G** in Schritt (a) des Verfahrens gemäß der Erfindung eingesetzt werden.

"Als Gemisch **G** in Schritt (a) des Verfahrens gemäß der Erfindung eingesetzt werden" bedeutet insbesondere, dass die beiden Ströme **S_{AB}** und **S_{BB}** in die Rektifikationskolonne **RD_{A}** geleitet werden, wobei sie bevorzugt vorher vermischt werden.

Sie können alternativ aber auch an zwei verschiedenen Zulaufstellen in die Rektifikationskolonne **RD_{A}** geleitet werden.

### 4.3 Bevorzugter Aspekt: Verfahren zur Umalkoholisierung eines Alkalimetallalkoholats

In einer vorteilhaften Ausführungsform der vorliegenden Erfindung wird die Energie, die in mindestens einem der Ströme **S_{OA1}, S_{OA2}, S_{OA11}, S_{OA12}** umfasst ist, für den Betrieb anderer industrieller Verfahren verwendet. Dies ist insbesondere in Verbundstandorten (Chemieparks, Technologieparks) vorteilhaft, in denen stets Bedarf für Heizwärme besteht. Gerade bei Verbunden mit mehreren Anlagen zur Alkalimetallalkoholatherstellung kann diese Energie vorteilhaft genutzt werden. Solch Verbunde umfassen typischerweise auch Verfahren zur Umalkoholisierung, wie sie in DE 27 26 491 A1 beschrieben ist. US 3,418,383 A beschreibt Verfahren zur Umalkoholatisierung aus Methanolaten zu Propylaten.

In einem bevorzugten Aspekt der vorliegenden Erfindung wird im Verfahren gemäß der vorliegenden Erfindung in einer Reaktivrektifikationskolonne **RR_{C}** ein Eduktstrom **S_{CE1}** umfassend M_{c}OR' und gegebenenfalls R'OH mit einem Eduktstrom **S_{CE2}** umfassend R"OH im Gegenstrom zu einem Rohprodukt **RP_{C}** umfassend M_{C}OR" und R'OH umgesetzt,
wobei am unteren Ende von **RR_{C}** ein Sumpfproduktstrom **S_{CP}** umfassend M_{C}OR" entnommen wird und am oberen Ende von **RR_{C}** ein Brüdenstrom **S_{CB}** umfassend R'OH entnommen wird,
und wobei R' und R" zwei voneinander verschiedene C₁ bis C₆-Kohlenwasserstoffreste sind, und M_{C} ein Metall ausgewählt aus Natrium, Kalium, bevorzugt Natrium, ist,
und wobei Energie von mindestens einem Teil eines Stroms ausgewählt aus **S_{OA1}, S_{OA2}** auf das Rohprodukt **RP_{C}** übertragen wird.

Das erfindungsgemäße Verfahren gemäß dem bevorzugten Aspekt der Erfindung ist eines zur Umalkoholisierung eines gegebenen Alkalimetallalkoholats M_{c}OR' zu einem anderen Alkalimetallalkoholat M_{c}OR", wie es beispielsweise in der DE 27 26 491 A1 beschrieben ist.

R' und R" sind dabei zwei voneinander verschiedene C₁ bis C₆-Kohlenwasserstoffreste, bevorzugt zwei voneinander verschiedene C₁ bis C₄-Kohlenwasserstoffreste.

Noch bevorzugter ist R' Methyl und R" ein C₂ bis C₄-Kohlenwasserstoffrest, noch bevorzugter ist R' = Methyl und R" = Ethyl.

Das Verfahren gemäß dem bevorzugten Aspekt der Erfindung (im Folgenden auch als "Umalkoholisierung" bezeichnet) wird in einer Reaktivrektifikationskolonne **RR_{C}** durchgeführt. Als Reaktivrektifikationskolonnen eignen sich Kolonnen, wie sie unter Punkt 4.2.1 im Kontext des Schrittes (α1) für **RR_{A}** beschrieben sind.

Die Reaktionskolonne **RR_{C}** wird mit oder ohne, vorzugsweise mit Rücklauf betrieben. Wird ein Rücklauf eingestellt, wird insbesondere der Brüden **S_{CB}** teilweise oder vollständig über einen Kondensator **K_{RRC}** geleitet, und der kondensierte Brüden kann dann wieder der Reaktionskolonne **RR_{C}** zugeführt werden oder als Eduktstrom **S_{AE1}** oder **S_{BE1},** eingesetzt werden. Er kann auch als Frisch-Alkohol Strom in **RD_{A}** eingesetzt werden.

Bei der Umalkoholisierung wird am unteren Ende von **RR_{C}** ein Sumpfproduktstrom **S_{CP}** umfassend M_{C}OR" entnommen. Am oberen Ende von **RR_{C}** wird ein Brüdenstrom **S_{CB}** umfassend R'OH entnommen.

Als Eduktstrom **S_{CE1}** umfassend M_{c}OR' und gegebenenfalls R'OH wird bevorzugt in den Ausführungsformen der Umalkoholisierung, in denen auch das Verfahren zu Herstellung eines Alkalimetallalkoholats gemäß der Erfindung durchgeführt wird, bevorzugt mindestens ein Teil von **S_{AP}** eingesetzt, insbesondere da R = Methyl ist, wodurch dann auch R' = Methyl ist. Besonders bevorzugt ist dann R" = Ethyl. Es findet demnach eine Umalkoholisierung von Alkalimetallmethanolat zum entsprechenden Alkalimetallethanolat statt.

Als Eduktstrom **S_{CE1}** umfassend M_{c}OR' und gegebenenfalls R'OH wird in einer bevorzugten Alternative in den Ausführungsformen der Umalkoholisierung, in denen auch das Verfahren zu Herstellung eines Alkalimetallalkoholats inklusive Schritt (α2) gemäß der Erfindung durchgeführt wird, bevorzugt mindestens ein Teil von **S_{BP}** eingesetzt, insbesondere da R = Methyl ist, wodurch dann auch R' = Methyl ist. Besonders bevorzugt ist dann R" = Ethyl. Es findet demnach eine Umalkoholisierung von Alkalimetallmethanolat zum entsprechenden Alkalimetallethanolat statt.

Wenn **S_{BP}** und **S_{AP}** das gleiche Alkalimetallalkoholat und den gleichen Alkohol ROH umfassen, können diese beiden Ströme auch getrennt oder gemischt als **S_{CE1}** eingesetzt werden, das heißt insbesondere erst vermischt und dann der Kolonne **RR_{C}** als Eduktstrom **S_{CE1}** zugeleitet werden oder getrennt der Kolonne **RR_{C}** als zwei Eduktströme **S_{CE1}** zugeleitet werden.

Der Eduktstrom **S_{CE2}** umfasst R"OH. In einer bevorzugten Ausführungsform liegt der Massenanteil von R"OH in **S_{CE2}** bei ≥ 85 Gew.-%, noch bevorzugter bei ≥ 90 Gew.-%, wobei **S_{CE2}** ansonsten insbesondere M_{C}OR" oder ein anderes Vergällungsmittel aufweist. Der als Eduktstrom **S_{CE2}** eingesetzte Alkohol R"OH kann auch handelsüblicher Alkohol mit einem Alkoholmassenanteil von mehr als 99.8 Gew.-% und einem Massenanteil an Wasser von bis zu 0.2 Gew.-% sein.

"Umsetzung eines Eduktstrom **S_{CE1}** umfassend M_{c}OR' und gegebenenfalls R'OH mit einem Eduktstrom **S_{CE2}** umfassend R"OH im Gegenstrom" wird erfindungsgemäß insbesondere dadurch gewährleistet, dass die Zulaufstelle mindestens eines Teils des Eduktstroms **S_{CE1}** umfassend M_{c}OR'an der Reaktionskolonne **RR_{C}** oberhalb der Zulaufstelle des Eduktstroms **S_{CE2}** umfassend R"OH liegt.

Die Reaktionskolonne **RR_{C}** wird mit oder ohne, vorzugsweise mit Rücklauf betrieben.

Die Reaktionskolonne **RR_{C}** umfasst in einer bevorzugten Ausführungsform mindestens einen Verdampfer, der insbesondere aus Zwischenverdampfern **V_{ZC}** und Sumpfverdampfern **V_{SC}** ausgewählt ist. Die Reaktionskolonne **RR_{C}** umfasst besonders bevorzugt mindestens einen Sumpfverdampfer **V_{SC}**.

Im Falle der Reaktionskolonne **RR_{C}** wird bei der Zwischenverdampfung mindestens ein Seitenstrom **S_{ZC}** aus **RR_{C}** entnommen ("abgezogen") und dem mindestens einen Zwischenverdampfer **V_{ZC}** zugeführt.

Im Falle der Reaktionskolonne **RR_{C}** wird bei der Sumpfverdampfung mindestens ein Strom, beispielsweise **S_{CP}** aus **RR_{C}** entnommen ("abgezogen") und mindestens ein Teil, im Falle von **S_{CP}** bevorzugt ein Teil, dem mindestens einen Sumpfverdampfer **V_{SC}** zugeführt.

Geeignete Verdampfer, die als Zwischenverdampfer und Sumpfverdampfer eingesetzt werden können, sind im Abschnitt 4.2.1 beschrieben.

Bei der Umalkoholisierung wird Energie, bevorzugt Wärme, von mindestens einem Teil eines Stroms ausgewählt aus **S_{OA1}, S_{OA2}** auf das Rohprodukt **RP_{C}** übertragen. Dies geschieht bevorzugt dadurch, dass Energie von mindestens einem Teil eines Stroms ausgewählt aus **S_{OA1}, S_{OA2}** auf **S_{CE1}** oder **S_{CE2}** bevor sie in **RR_{C}** geleitet werden, übertragen wird, und dann von **S_{CE1}** bzw. **S_{CE2}** auf das in **RR_{C}** befindliche Rohprodukt **RP_{C}**, mit dem sie sich mischen, übertragen wird.

Demnach wird Energie, bevorzugt Wärme, von mindestens einem Teil eines Stroms ausgewählt aus **S_{OA1}, S_{OA2},** insbesondere von mindestens einem Strom ausgewählt aus **S_{OA11}, S_{OA12}, S_{OA2},** bevorzugt von mindestens einem Strom ausgewählt aus **S_{OA11},** einem Teil von **S_{OA2}** auf das Rohprodukt **RP_{C}** übertragen.

"Übertragung von Energie, bevorzugt Wärme, von mindestens einem Teil von **S_{OA1}** auf das Rohprodukt **RP_{C}**" umfasst auch die Übertragung von Energie, bevorzugt Wärme, von mindestens einem Strom ausgewählt aus **S_{OA11}, S_{OA12},** Strom **S_{OA1}** vor dessen Auftrennung in **S_{OA11}, S_{OA12},** auf das Rohprodukt **RP_{C}**.

Daneben kann auch Rohprodukt **RP_{C}** über einen Zwischenverdampfer **V_{ZC}** oder einen Sumpfverdampfer **V_{SC}** geleitet werden und in **V_{ZC}** bzw. **V_{SC}** Energie, bevorzugt Wärme, von mindestens einem Teil eines Stroms ausgewählt aus **S_{OA1}, S_{OA2}** auf das Rohprodukt **RP_{C}** übertragen werden.

Daneben kann auch der Sumpfproduktstrom **S_{CP}** teilweise über einen Sumpfverdampfer **V_{SC}** geleitet und dann teilweise wieder in **RR_{C}** rückgeführt werden, wobei in **V_{SC}** Energie, bevorzugt Wärme, von mindestens einem Teil eines Stroms ausgewählt aus **S_{OA1}, S_{OA2}** auf den rückgeführten Teil von **S_{CP}** übertragen wird und dann, in der Kolonne **RR_{C}**, von **S_{CP}** auf in der Kolonne befindliches Rohprodukt **RP_{C}** übertragen wird.

Die Übertragung der Energie von mindestens einem Teil eines Stroms ausgewählt aus **S_{OA1}, S_{OA2}** auf die genannten Ströme erfolgt dabei direkt oder indirekt, das heißt ohne bzw. mit Wärmeträger **W₁**, entsprechend wie in Abschnitt 4.1.4 beschrieben.

Die bevorzugte Ausführungsform des erfindungsgemäßen Verfahrens ermöglicht es, die Energie aus **S_{OA1}, S_{OA2},** insbesondere aus **S_{OA2}, S_{OA11}, S_{OA12}** effizient einzusetzen. Dadurch sinkt der Gesamtenergiebedarf.

### 5. Beispiele

### 5.1 Beispiel 1 (nicht erfindungsgemäß), entspricht Abbildung 1:

Ein Strom wässriger NaOH (50 Gew.-%) **S_{AE2}** <102> von 100 kg/h wird mit 30 °C am Kopf einer Reaktionskolonne **RR_{A}** <100> zugeführt. Im Gegenstrom wird ein dampfförmiger Methanolstrom **S_{AE1}** <103> von 1034.9 kg/h am Sumpf der Reaktionskolonne **RR_{A}** <100> zugeführt. Die Reaktionskolonne **RR_{A}** <100> wird bei einem Kopfdruck von 2.15 bar abs. betrieben. Am Sumpf der Kolonne **RR_{A}** <100> wird ein nahezu wasserfreier Produktstrom **S_{AP*}** <104> von 219.7 kg/h entnommen (30 Gew.-% Natriummethanolat in Methanol). Am Verdampfer **V_{SA}** <105> der Reaktionskolonne **RR_{A}** <100> werden ca. 24 kW Heizleistung mit Hilfe von Niederdruckdampf eingespeist. Ein dampfförmiger Methanol-Wasser-Strom **S_{AB}** <107> wird am Kopf der Reaktionskolonne **RR_{A}** <100> entnommen, davon werden 80 kg/h im Kondensator **K_{RRA}** <108> kondensiert und als Rücklauf auf die Reaktionskolonne **RR_{A}** <100> zurückgefahren, der verbleibende Strom von 915.2 kg/h wird einer Rektifikationskolonne **RD_{A}** <300> zugeführt. Die Rektifikationskolonne **RD_{A}** <300> wird bei einem Kopfdruck von 2.0 bar abs. betrieben. Am Sumpf der Rektifikationskolonne **RD_{A}** <300> wird ein flüssiger Wasserstrom **S_{UA}** <304> von 72.2 kg/h abgeführt (500 Gew.-ppm Methanol). Am Kopf der Rektifikationskolonne **RD_{A}** <300> wird ein dampfförmiger Methanolstrom **S_{OA}** <302> (2 bar, 83 °C; 200 Gew.-ppm Wasser) von 1903.6 kg/h entnommen, davon werden 63.9 kg/h in einem Kondensator **K_{RD}** <407> kondensiert, der verbleibende Strom wird einem ersten Verdichter **VD_{AB2}** <303> zugeführt und dort auf 2.6 bar abs. verdichtet. Anschließend wird der Strom aufgeteilt, ein Strom von 1034.9 kg/h wird zu der Reaktionskolonne **RR_{A}** <100> zurückgeführt. Der Rest von 804.8 kg/h wird einer mehrstufigen Verdichtung mit Zwischenkühlung zugeführt. Im Verdichter **VD₁** <401> wird der Strom auf **p_{OA1}** = 4.8 bar abs. und **T_{OA1}** = 156 °C verdichtet, man erhält Strom <403>. In der anschließenden Zwischenkühlung im Zwischenkühler **WT_{X}** <402> wird der Strom auf 145 °C abgekühlt, wobei rund 4.4 kW an Wärme über Kühlwasser abgeführt werden. Im Verdichter **VD_{X}** <405> wird der Strom schließlich weiter auf 9.0 bar und 200 °C verdichtet, man erhält Strom <404>. Im nachfolgenden Kondensator, der zugleich der Sumpfverdampfer **V_{SRD}** <406> der Rektifikationskolonne **RD_{A}** <300> ist, werden die ca. 238 kW an Heizleistung für die Rektifikationskolonne **RD_{A}** <300> zur Verfügung gestellt. Der dabei kondensierende Methanolstrom <404> wird zusammen mit 191.9 kg/h Frischmethanol (1000 Gew.-ppm Wasser) <408> und den 63.9 kg/h zuvor kondensierten Brüden gemischt und zurück auf den Kopf der Rektifikationskolonne **RD_{A}** <300> aufgegeben.

Die Verdichterleistung beträgt in Summe rund 55 kW.

Zusammen mit den 24 kW für den Heizdampf ergibt sich ein Leistungsbedarf von rund 79 kW für Verdichter und Heizdampf.

### 5.2 Beispiel 2 (nicht erfindungsgemäß), entspricht Abbildung 2:

Die Anordnung im nicht erfindungsgemäßen Beispiel 2 entspricht jenem gemäß Beispiel 1 mit folgenden Unterschieden:
Die Rektifikationskolonne **RD_{A}** <300> weist einen Zwischenverdampfer **V_{ZRD}** <409> auf. Dabei wird der Rektifikationskolonne **RD_{A}** <300> ein Flüssigkeitsstrom **S_{ZA}** <305> mit 94 °C entnommen und auf diesen in dem Zwischenverdampfer **V_{ZRD}** <409> rund 230 kW Wärme übertragen, wobei der Strom teilweise verdampft und anschließend wieder der Rektifikationskolonne **RD_{A}** <300> zugeführt wird.

Am Kopf der Rektifikationskolonne **RD_{A}** <300> wird ein dampfförmiger Methanolstrom **S_{OA}** <302> (200 Gew.-ppm Wasser) von 1887.1 kg/h entnommen, davon werden 89.4 kg/h in einem Kondensator **K_{RD}** <407> kondensiert. Der verbleibende Strom wird wie im Beispiel 1 in einem ersten Verdichter **VD_{AB2}** <303> auf 2.6 bar abs. verdichtet. Anschließend wird ein Teilstrom von 1034.9 kg/h zur Reaktionskolonne **RR_{A}** <100> zurückgeführt. Der Rest von 762.8 kg/h wird auf 5.6 bar abs. und 168 °C verdichtet, man erhält Strom <403>. Im nachfolgenden Kondensator, der zugleich der Zwischenverdampfer **V_{ZRD}** <409> der Rektifikationskolonne **RD_{A}** <300> ist, werden die ca. 230 kW an Heizleistung für die Rektifikationskolonne **RD_{A}** <300> zur Verfügung gestellt. Der dabei kondensierende Methanolstrom <403> wird zusammen mit 191.9 kg/h Frischmethanol <408> und den 89.4 kg/h zuvor kondensierten Brüden gemischt und zurück auf den Kopf der Rektifikationskolonne **RD_{A}** <300> aufgegeben. Am Sumpfverdampfer **V_{SRD}** <406> der Rektifikationskolonne **RD_{A}** <300> werden ca. 20 kW Heizleistung mit Hilfe von Niederdruckdampf eingetragen. Gegenüber Beispiel 1 muss der Brüdenstrom nicht auf 9 bar abs., sondern nur auf 5.6 bar abs. verdichtet werden, um die Wärme an den Verdampfer **V_{ZRD}** <409> abgeben zu können, da die Siedetemperatur im Zwischenverdampfer **V_{ZRD}** <409> geringer als im Sumpfverdampfer **V_{SRD}** <406> ist.

Die Verdichterleistung beträgt in Summe somit nur rund 38 kW (statt 55 kW), dafür erhört sich der Heizdampfbedarf gegenüber Beispiel 1 auf rund 44 kW, da im Sumpfverdampfer **V_{SRD}** <406> eine Heizleistung von 20 kW erforderlich ist und diese mit Wärme mithilfe von Niederdruckdruck eingetragen wird.

Der Leistungsbedarf für Verdichter und Heizdampf beträgt in Summe somit rund 82 kW.

### 5.3 Beispiel 3 (erfindungsgemäß), entspricht Abbildung 3:

Die Anordnung im erfindungsgemäßen Beispiel 3 entspricht jener gemäß den Beispielen 1 und 2 mit folgenden Unterschieden:
Am Kopf der Rektifikationskolonne **RD_{A}** <300> wird ein dampfförmiger Methanolstrom **S_{OA}** <302> (200 Gew.-ppm Wasser) von 1898.9 kg/h entnommen, davon werden 33.9 kg/h in einem Kondensator **K_{RD}** <407> kondensiert. Der verbleibende Strom wird wie im Beispiel 2 in einem ersten Verdichter **VD_{AB2}** <303> auf 2.6 bar abs. verdichtet und anschließend ein Teilstrom von 1034.9 kg/h zur Reaktionskolonne **RR_{A}** <100> zurückgeführt. Der Rest von 830.1 kg/h wird zunächst auf 5.6 bar abs. und 169 °C verdichtet, wodurch man Strom **S_{OA1}** <403> erhält. Von diesem Strom wird ein Teil **S_{OA11}** <4031> (761.7 kg/h) in einen nachfolgenden Kondensator geleitet, der zugleich der Zwischenverdampfer **V_{ZRD}** <409> der Rektifikationskolonne **RD_{A}** <300> ist, und ca. 230 kW an Heizleistung für die Rektifikationskolonne **RD_{A}** <300> zur Verfügung gestellt. Der andere Teil **S_{OA12}** <4032> (68.4 kg/h) wird anschließenden in einer Zwischenkühlung im Zwischenkühler **WT_{X}** <402> auf ca. 154°C abgekühlt, wobei ca. 0.5 kW an Wärme über Kühlwasser abgeführt werden. Anschließend wird der Strom **S_{OA12}** <4032> in einem weiteren Verdichter **VDₓ** <405> verdichtet, man erhält Strom **S_{OA2}** <404> mit **p_{OA2}** = 9.0 bar und **T_{OA2}** = 196 °C. Im nachfolgenden Kondensator, der zugleich der Sumpfverdampfer **V_{SRD}** <406> der Rektifikationskolonne **RD_{A}** <300> ist, werden ca. 20 kW an Heizleistung für die Rektifikationskolonne **RD_{A}** <300> zur Verfügung gestellt. Die im Zwischen- und Sumpfverdampfer **V_{ZRD}** <409> und **V_{SRD}** <406> kondensierten Methanolströme **S_{OA11}** <4031> und **S_{OA2}** <404> werden zusammen mit 191.9 kg/h Frischmethanol <408> und den 33.9 kg/h zuvor kondensierten Brüden gemischt und zurück auf den Kopf der Rektifikationskolonne **RD_{A}** <300> aufgegeben.

Der Verdichterleistung beträgt in Summe rund 42 kW (statt 55 kW in Beispiel 1). Da kein Niederdruckdampf für den Sumpfverdampfer **V_{SRD}** <406> erforderlich ist, müssen wie in Beispiel 1 nur rund 24 kW mittels Heizdampf zur Verfügung gestellt werden. Der Leistungsbedarf für Verdichter und Heizdampf sinkt in Summe somit auf rund 66 kW.

Gegenüber Beispiel 1 muss nur ein geringerer Brüdenstrom auf 9 bar abs. verdichtet werden, ein Großteil des Brüdenstroms muss wie in Beispiel 2 nur auf 5.6 bar abs. verdichtet werden, so dass die Gesamt-Verdichterleistung sinkt. Gegenüber Beispiel 2 ist im stationären Betrieb jedoch kein Niederdruckdampf im Sumpfverdampfer **V_{SRD}** <406> erforderlich, sodass der Heizdampfbedarf gegenüber Beispiel 2 sinkt.

Die aufzuwendende Gesamtenergie wird durch das Verfahren gemäß Beispiel 3 minimiert.

Der jeweils nötige Anteil an Heizleistung durch Niederdruckdampf und Verdichterleistung ist in Abbildung 10 gezeigt.

**Ergebnis:** Durch die erfindungsgemäße Vorgehensweise, den Brüdenstrom stufenweise zu verdichten und damit den Zwischenverdampfer und Sumpfverdampfer mit dem unterschiedlich verdichteten Brüden zu betreiben, kann überraschenderweise Energie eingespart werden.

## Patentansprüche

1. Verfahren zur Herstellung mindestens eines Alkalimetallalkoholats der Formel M_{A}OR, wobei R Methyl ist, und wobei M_{A} ein Metall ausgewählt aus Natrium, Kalium, ist, wobei:
(α1) ein Eduktstrom **S_{AE1}** umfassend ROH mit einem Eduktstrom **S_{AE2}** umfassend M_{A}OH im Gegenstrom in einer Reaktivrektifikationskolonne **RR_{A}** zu einem Rohprodukt **RP_{A}** umfassend M_{A}OR, Wasser, ROH, M_{A}OH umgesetzt wird,
wobei am unteren Ende von **RR_{A}** ein Sumpfproduktstrom **S_{AP}** umfassend ROH und M_{A}OR entnommen wird und am oberen Ende von **RR_{A}** ein Brüdenstrom **S_{AB}** umfassend Wasser und ROH entnommen wird,
(α2) und gegebenenfalls, gleichzeitig mit und räumlich getrennt von Schritt (α1), ein Eduktstrom **S_{BE1}** umfassend ROH mit einem Eduktstrom **S_{BE2}** umfassend M_{B}OH im Gegenstrom in einer Reaktivrektifikationskolonne **RR_{B}** zu einem Rohprodukt **RP_{B}** umfassend M_{B}OR, Wasser, ROH, M_{B}OH umgesetzt wird, wobei M_{B} ein Metall ausgewählt aus Natrium, Kalium ist,
wobei am unteren Ende von **RR_{B}** ein Sumpfproduktstrom **S_{BP}** umfassend ROH und M_{B}OR entnommen wird und am oberen Ende von **RR_{B}** ein Brüdenstrom **S_{BB}** umfassend Wasser und ROH entnommen wird,
(β) mindestens ein Teil des Brüdenstroms **S_{AB}**, und, wenn Schritt (α2) durchgeführt wird, mindestens ein Teil des Brüdenstrom **S_{BB}**, vermischt mit **S_{AB}** oder getrennt von **S_{AB}**, als Gemisch **G** in Schritt (a) eines Verfahrens zur Aufarbeitung eines Gemisches **G** umfassend Wasser und Alkohol ROH eingesetzt wird,
wobei in dem Verfahren zur Aufarbeitung eines Gemisches **G**
(a) das Gemisch **G** in eine Rektifikationskolonne **RD_{A}** geleitet wird und in **RD_{A}** in mindestens einen Brüdenstrom **S_{OA}** umfassend ROH, der am oberen Ende von **RD_{A}** entnommen wird, und mindestens einen Strom **S_{UA}** umfassend Wasser, der am unteren Ende von **RD_{A}** entnommen wird, aufgetrennt wird,
(b) mindestens ein Seitenstrom **S_{ZA}** aus **RD_{A}** entnommen wird und wieder in **RD_{A}** rückgeführt wird,
(c) mindestens ein Teil von **S_{OA}** verdichtet wird, wodurch ein gegenüber **S_{OA}** verdichteter Brüdenstrom **S_{OA1}** erhalten wird,
(d) Energie von einem ersten Teil **S_{OA11}** des verdichteten Brüdenstroms **S_{OA1}** auf **S_{ZA}** übertragen wird, bevor **S_{ZA}** in **RD_{A}** rückgeführt wird,
(e) ein von **S_{OA11}** verschiedener Teil des verdichteten Brüdenstroms **S_{OA1}, S_{OA12},** weiter verdichtet wird, wodurch ein gegenüber **S_{OA11}** verdichteter Brüdenstrom **S_{OA2}** erhalten wird,
(f) Energie von mindestens einem Teil von **S_{OA2}** auf mindestens einen Teil **S_{UA1}** von **S_{UA}** übertragen wird, bevor **S_{UA1}** in **RD_{A}** rückgeführt wird.

2. Verfahren nach Anspruch 1, wobei in Schritt (d) Energie von **S_{OA11}** auf **S_{ZA}** in einem Zwischenverdampfer **V_{ZRD}** übertragen wird.

3. Verfahren nach Anspruch 1 oder 2, wobei in Schritt (f) Energie von mindestens einem Teil von **S_{OA2}** auf den mindestens einen Teil **S_{UA1}** von **S_{UA}** in einem Sumpfverdampfer **V_{SRD}** übertragen wird.

4. Verfahren nach einem der Ansprüche 1 bis 3, wobei Energie von **S_{OA11},** nachdem Energie von **S_{OA11}** auf **S_{ZA}** gemäß Schritt (d) übertragen wurde, auf **S_{OA}** übertragen wird, und/oder Energie von mindestens einen Teil von **S_{OA2},** nachdem Energie von diesem auf den mindestens einen Teil **S_{UA1}** von **S_{UA}** gemäß Schritt (f) übertragen wurde, auf **S_{OA}** übertragen wird.

5. Verfahren nach einem der Ansprüche 1 bis 4, wobei mindestens zwei der Kolonnen ausgewählt aus Rektifikationskolonne **RD_{A},** Reaktionskolonne **RR_{A}** und, falls Schritt (α2) durchgeführt wird, Reaktionskolonne **RR_{B}** in einem Kolonnenmantel untergebracht sind, wobei die Kolonnen mindestens teilweise durch eine bis zum Boden der Kolonne durchgezogene Trennwand voneinander getrennt sind.

6. Verfahren nach einem der Ansprüche 1 bis 5, wobei ein Teil von **S_{OA}** in Schritt (α1) als Eduktstrom **S_{AE1}** und, falls Schritt (α2) durchgeführt wird, alternativ oder zusätzlich in Schritt (α2) als Eduktstrom **S_{BE1}** eingesetzt wird.

7. Verfahren nach einem der Ansprüche 1 bis 6, wobei Energie von mindestens einem Teil eines Stroms ausgewählt aus **S_{OA1}, S_{OA2}** auf das Rohprodukt **RP_{A}** und, falls Schritt (α2) durchgeführt wird, alternativ oder zusätzlich auf das Rohprodukt **RP_{B}** übertragen wird.

8. Verfahren nach einem der Ansprüche 1 bis 7, wobei in einer Reaktivrektifikationskolonne **RR_{C}** ein Eduktstrom **S_{CE1}** umfassend M_{c}OR' mit einem Eduktstrom **S_{CE2}** umfassend R"OH im Gegenstrom zu einem Rohprodukt **RP_{C}** umfassend M_{C}OR" und R'OH umgesetzt wird,
wobei am unteren Ende von **RR_{C}** ein Sumpfproduktstrom **S_{CP}** umfassend M_{C}OR" entnommen wird und am oberen Ende von **RR_{C}** ein Brüdenstrom **S_{CB}** umfassend R'OH entnommen wird,
und wobei R' und R" zwei voneinander verschiedene C₁ bis C₆-Kohlenwasserstoffreste sind, und M_{C} ein Metall ausgewählt aus Natrium, Kalium ist,
und wobei Energie von mindestens einem Teil eines Stroms ausgewählt aus **S_{OA1}, S_{OA2}** auf das Rohprodukt **RP_{C}** übertragen wird.

9. Verfahren nach Anspruch 8, wobei R' = Methyl ist.

10. Verfahren nach Anspruch 9, wobei **S_{AP}** nach dem Verfahren nach Anspruch 1 bis 7 erhalten wird, wobei R = Methyl ist, und wobei mindestens ein Teil von **S_{AP}** als **S_{CE1}** eingesetzt wird.

11. Verfahren nach Anspruch 9, wobei **S_{BP}** nach dem Verfahren nach Anspruch 1 bis 7 unter Durchführung des Schrittes (α2) erhalten wird, wobei R = Methyl ist, und wobei mindestens ein Teil von **S_{BP}** als **S_{CE1}** eingesetzt wird.

12. Verfahren nach einem der Ansprüche 9 bis 11, wobei R" = Ethyl ist.

## Claims

1. Process for producing at least one alkali metal alkoxide of formula M_{A}OR, wherein R is methyl, and wherein M_{A} is a metal selected from sodium, potassium, wherein:
(α1) a reactant stream **S_{AE1}** comprising ROH is reacted with a reactant stream **S_{AE2}** comprising M_{A}OH in countercurrent in a reactive rectification column **RR_{A}** to afford a crude product **RP_{A}** comprising M_{A}OR, water, ROH, M_{A}OH,
wherein a bottoms product stream **S_{AP}** comprising ROH and M_{A}OR is withdrawn at the lower end of **RR_{A}** and a vapour stream **S_{AB}** comprising water and ROH is withdrawn at the upper end of **RR_{A}**,
(α2) and optionally, simultaneously with and spatially separate from step (α1), a reactant stream **S_{BE1}** comprising ROH is reacted with a reactant stream **S_{BE2}** comprising M_{B}OH in countercurrent in a reactive rectification column **RR_{B}** to afford a crude product **RP_{B}** comprising M_{B}OR, water, ROH, M_{B}OH, wherein M_{B} is a metal selected from sodium, potassium,
wherein a bottoms product stream **S_{BP}** comprising ROH and M_{B}OR is withdrawn at the lower end of **RR_{B}** and a vapour stream **S_{BB}** comprising water and ROH is withdrawn at the upper end of **RR_{B},**
(β) at least a portion of the vapour stream **S_{AB}**, and if step (α2) is performed at least a portion of the vapour stream **S_{BB}**, in admixture with **S_{AB}** or separate from **S_{AB}**, is employed as mixture **G** in step (a) of a process for workup of a mixture **G** comprising water and alcohol ROH,
wherein in the process for workup of a mixture **G**
(a) the mixture **G** is passed into a rectification column **RD_{A}** and in **RD_{A}** separated into at least one vapour stream **S_{OA}** comprising ROH which is withdrawn at the upper end of **RD_{A}** and at least one stream **S_{UA}** comprising water which is withdrawn at the lower end of **RD_{A},**
(b) at least one side stream **S_{ZA}** is withdrawn from **RD_{A}** and recycled to **RD_{A},**
(c) at least a portion of **S_{OA}** is compressed to afford a vapour stream **S_{OA1}** compressed relative to **S_{OA}**,
(d) energy is transferred from a first portion **S_{OA11}** of the compressed vapour stream **S_{OA1}** to **S_{ZA}** before **S_{ZA}** is recycled to **RD_{A},**
(e) a portion **S_{OA12}** of the compressed vapour stream **S_{OA1}** that is distinct from **S_{OA11}** is subjected to further compression to afford a vapour stream **S_{OA2}** that is compressed relative to **S_{OA11},**
(f) energy is transferred from at least a portion of **S_{OA2}** to at least a portion **S_{UA1}** of **S_{UA}** before **S_{UA1}** is recycled to **RD_{A}**.

2. Process according to Claim 1, wherein in step (d) energy is transferred from **S_{OA11}** to **S_{ZA}** in an intermediate evaporator **V_{ZRD}.**

3. Process according to Claim 1 or 2, wherein in step (f) energy is transferred from at least a portion of **S_{OA2}** to the at least a portion **S_{UA1}** of **S_{UA}** in a bottoms evaporator **V_{SRD}**.

4. Process according to any of Claims 1 to 3, wherein once energy has been transferred from **S_{OA11}** to **S_{ZA}** according to step (d) energy is transferred from **S_{OA11}** to **S_{OA}** and/or once energy has been transferred from at least a portion of **S_{OA2}** to the at least a portion **S_{UA1}** of **S_{UA}** according to step (f) energy is transferred from at least a portion of **S_{OA2}** to **S_{OA}**.

5. Process according to any of Claims 1 to 4, wherein at least two of the columns selected from rectification column **RD_{A},** reaction column **RR_{A}** and if step (α2) is performed reaction column **RR_{B}** are accommodated in one column shell, wherein the columns are at least partially separated from one another by a dividing wall extending to the bottom of the column.

6. Process according to any of Claims 1 to 5, wherein a portion of **S_{OA}** is employed as reactant stream **S_{AE1}** in step (α1) and if step (α2) is performed alternatively or in addition as reactant stream **S_{BE1}** in step (α2).

7. Process according to any of Claims 1 to 6, wherein energy is transferred from at least a portion of a stream selected from **S_{OA1}**, **S_{OA2}** to the crude product **RP_{A}** and if step (α2) is performed alternatively or in addition to the crude product **RP_{B}.**

8. Process according to any of Claims 1 to 7, wherein in a reactive rectification column **RR_{C}** a reactant stream **S_{CE1}** comprising M_{c}OR' is reacted in countercurrent with a reactant stream **S_{CE2}** comprising R"OH to afford a crude product **RP_{C}** comprising M_{C}OR" and R'OH,
wherein a bottoms product stream **S_{CP}** comprising M_{C}OR" is withdrawn at the lower end of **RR_{C}** and a vapour stream **S_{CB}** comprising R'OH is withdrawn at the upper end of **RR_{C}**,
and wherein R' and R" are two distinct C₁ to C₆ hydrocarbon radicals and M_{C} is a metal selected from sodium, potassium,
and wherein energy is transferred from at least a portion of a stream selected from **S_{OA1}**, **S_{OA2}** to the crude product **RP_{C}.**

9. Process according to Claim 8, wherein R' = methyl.

10. Process according to Claim 9, wherein the process according to Claims 1 to 7 affords **S_{AP},** wherein R = methyl and wherein at least a portion of **S_{AP}** is employed as **S_{CE1}.**

11. Process according to Claim 9, wherein the process according to Claims 1 to 7 with performance of step (α2) affords **S_{BP}**, wherein R = methyl and wherein at least a portion of **S_{BP}** is employed as **S_{CE1}.**

12. Process according to any of Claims 9 to 11, wherein R" = ethyl.

## Revendications

1. Procédé de fabrication d'au moins un alcoolate d'un métal alcalin de formule M_{A}OR, dans laquelle R représente un méthyle, et M_{A} est un métal choisi parmi le sodium, le potassium, dans lequel :
(α1) un courant de réactifs **S_{AE1}** comprenant du ROH avec un courant de réactifs **S_{AE2}** comprenant du M_{A}OH est mis à réagir à contre-courant dans une colonne de rectification réactive **RR_{A}** pour obtenir un produit brut **RP_{A}** comprenant du M_{A}OR, de l'eau, du ROH, du M_{A}OH,
dans lequel, à l'extrémité inférieure de **RR_{A}**, un courant de produit de fond **S_{AP}** comprenant du ROH et du M_{A}OR est soutiré, et à l'extrémité supérieure de **RR_{A}**, un courant de vapeur **S_{AB}** comprenant de l'eau et du ROH est soutiré,
(α2) et éventuellement, en même temps que l'étape (α1) et spatialement séparément de cette dernière, un courant de réactifs **S_{BE1}** comprenant du ROH avec un courant de réactifs **S_{BE2}** comprenant du M_{B}OH est mis à réagir à contre-courant dans une colonne de rectification réactive **RR_{B}** pour obtenir un produit brut **RP_{B}** comprenant du M_{B}OR, de l'eau, du ROH, du M_{B}OH, M_{B} étant un métal choisi parmi le sodium, le potassium,
dans lequel, à l'extrémité inférieure de **RR_{B},** un courant de produit de fond **S_{BP}** comprenant du ROH et du M_{B}OR est soutiré et à l'extrémité supérieure de **RR_{B},** un courant de vapeur **S_{BB}** comprenant de l'eau et du ROH est soutiré, (β) au moins une partie du courant de vapeur **S_{AB}** et, lors de la mise en œuvre de l'étape (α2), au moins une partie du courant de vapeur **S_{BB}**, en mélange avec **S_{AB}** ou séparément de **S_{AB}**, sont utilisées comme un mélange **G** dans l'étape (a) d'un procédé de traitement d'un mélange **G** comprenant de l'eau et un alcool ROH,
dans lequel, dans le procédé de traitement d'un mélange **G**
(a) le mélange **G** est envoyé dans une colonne de rectification **RD_{A}** et, dans **RD_{A},** est séparé en au moins un courant de vapeur **S_{OA}** comprenant du ROH, qui est soutiré à l'extrémité supérieure de **RD_{A},** et en au moins un courant **S_{UA}** comprenant de l'eau, qui est soutiré à l'extrémité inférieure de **RD_{A},**
(b) au moins un courant latéral **S_{ZA}** est soutiré de **RD_{A}** et renvoyé dans **RD_{A},**
(c) au moins une partie de **S_{OA}** est comprimée, ce qui donne un courant de vapeur **S_{OA1}** comprimé par rapport à **S_{OA},**
(d) l'énergie d'une première partie **S_{OA11}** du courant de vapeur comprimé **S_{OA1}** est transférée à **S_{ZA}** avant renvoi de **S_{ZA}** dans **RD_{A},**
(e) une partie différente de **S_{OA11}** du courant de vapeur comprimé **S_{OA1}**, **S_{OA12},** subit une compression plus poussée, ce qui donne un courant de vapeur **S_{OA2}** comprimé par rapport à **S_{OA11}**,
(f) l'énergie d'au moins une partie de **S_{OA2}** est transférée à au moins une partie **S_{UA1}** de **S_{UA}** avant renvoi de **S_{UA1}** dans **RD_{A}**.

2. Procédé selon la revendication 1, dans lequel dans l'étape (d), l'énergie de **S_{OA11}** est transférée à **S_{ZA}** dans un évaporateur intermédiaire **V_{ZRD}.**

3. Procédé selon la revendication 1 ou 2, dans lequel dans l'étape (f), l'énergie d'au moins une partie de **S_{OA2}** est transférée à au moins une partie **S_{UA1}** de **S_{UA}** dans un évaporateur de fond **V_{SRD}.**

4. Procédé selon l'une des revendications 1 à 3, dans lequel l'énergie de **S_{OA11},** après que l'énergie de **S_{OA11}** a été transférée à **S_{ZA}** selon l'étape (d), est transférée à **S_{OA}**, et/ou l'énergie d'au moins une partie de **S_{OA2}**, après que l'énergie de cette dernière a été transférée à au moins une partie **S_{UA1}** de **S_{UA}** selon l'étape (f), est transférée à **S_{OA}.**

5. Procédé selon l'une des revendications 1 à 4, dans lequel au moins deux des colonnes choisies parmi la colonne de rectification **RD_{A},** la colonne de réaction **RR_{A}** et, si l'étape (α2) est mise en œuvre, la colonne de réaction **RR_{B}**, sont disposées dans une chemise de colonne, les colonnes étant au moins partiellement séparées les unes des autres par une cloison jusqu'au fond de la colonne.

6. Procédé selon l'une des revendications 1 à 5, dans lequel une partie de **S_{OA}** est utilisée dans l'étape (α1) comme courant de réactifs **S_{AE1}** et, si l'étape (α2) est mise en œuvre, est utilisée en alternance ou en plus dans l'étape (α2) comme courant de réactifs **S_{BE1}.**

7. Procédé selon l'une des revendications 1 à 6, dans lequel l'énergie d'au moins une partie d'un courant choisi parmi **S_{OA1}, S_{OA2},** est transférée au produit brut **RP_{A}** et, si l'étape (α2) est mise en œuvre, est transférée en alternance ou en outre au produit brut **RP_{B.}**

8. Procédé selon l'une des revendications 1 à 7, dans lequel, dans une colonne de rectification réactive **RR_{C}**, un courant de réactifs **S_{CE1}** comprenant du M_{C}OR' avec un courant de réactifs **S_{CE2}** comprenant du R"OH est mis à réagir à contre-courant pour obtenir un produit brut **RP_{C}** comprenant du M_{C}OR" et du R'OH,
dans lequel à l'extrémité inférieure de **RR_{C}**, un courant de produit de fond **S_{CP}** comprenant du M_{C}OR" est soutiré et à l'extrémité supérieure de **RR_{C}**, un courant de vapeur **S_{CB}** comprenant du R'OH est soutiré,
et dans lequel R' et R" représentent des radicaux hydrocarbonés en C₁ à C₆ différents l'un de l'autre, et M_{C} est un métal choisi parmi le sodium, le potassium,
et dans lequel l'énergie d'au moins une partie d'un courant choisi parmi **S_{OA1}**, **S_{OA2}**, est transférée au produit brut **RP_{C}**.

9. Procédé selon la revendication 8, dans lequel R' = méthyle.

10. Procédé selon la revendication 9, dans lequel **S_{AP}** est obtenu par le procédé selon les revendications 1 à 7, dans lequel R = méthyle, et dans lequel au moins une partie de **S_{AP}** est utilisée comme **S_{CE1}.**

11. Procédé selon la revendication 9, dans lequel **S_{BP}** est obtenu par le procédé selon les revendications 1 à 7 par mise en œuvre de l'étape (α2), dans lequel R = méthyle, et dans lequel au moins une partie de **S_{BP}** est utilisée comme **S_{CE1}.**

12. Procédé selon l'une des revendications 9 à 11, dans lequel R" = éthyle.
